# EUROPÄISCHE PATENTSCHRIFT

(11) **EP 3 470 364 B1**
(45) Veröffentlichungstag und Bekanntmachung des Hinweises auf die Patenterteilung: **20.08.2025**
(21) Anmeldenummer: 18199640.6
(22) Anmeldetag: 10.10.2018
(51) Int. Cl.: B67C 3/00, B67C 7/00, B65B 55/10, A61L 2/18, A61L 2/22, A61L 2/14

(54) **VERFAHREN ZUR DESINFEKTION VON KOMPONENTEN EINER ABFÜLLANLAGE UND ABFÜLLANLAGE**
PROCESS FOR THE DISINFECTION OF COMPONENTS OF A FILLING MACHINE, AND FILLING MACHINE
PROCÉDÉ DE DÉSINFECTION DE COMPOSANTS D'UNE INSTALLATION DE REMPLISSAGE ET INSTALLATION DE REMPLISSAGE

(30) Priorität: 10.10.2017 DE 102017123507; 13.10.2017 DE 102017123871
(43) Veröffentlichungstag der Anmeldung: 17.04.2019
(73) Patentinhaber: Plasmatreat GmbH, 33803 Steinhagen (DE)
(72) Erfinder: BUSKE, Christian, 33619 Bielefeld (DE); SCHMITT-JOHN, Prof.-Dr. Thomas, 33803 Steinhagen (DE); HASSE, Daniel, 33104 Paderborn (DE)
(74) Vertreter: Cohausz & Florack

(56) Entgegenhaltungen:
- EP-A1- 3 146 983
- WO-A1-2007/071720
- WO-A1-2014/161843
- WO-A1-2017/064741
- JP-A- 2001 054 556

## Beschreibung

Die Erfindung betrifft Verfahren zur Desinfektion einer Oberfläche sowie Verfahren zur Desinfektion von Komponenten einer Abfüllanlage und/oder von beim Betrieb der Abfüllanlage verwendeten Materialien. Weiterhin betrifft die Erfindung eine Abfüllanlage. Darüber hinaus betrifft die Erfindung ein Verfahren und eine Vorrichtung zur Herstellung von plasmaaktiviertem Wasser sowie eine Verwendung von plasmaaktiviertem Wasser.

Abfüllanlagen für Lebensmittel, zum Beispiel Getränkeabfüllanlagen, unterliegen strengen Hygienevorschriften. Insbesondere müssen bestimmte Komponenten der Abfüllanlagen regelmäßig desinfiziert werden. Gleiches gilt für die beim Betrieb der Abfüllanlagen verwendeten Materialien, insbesondere die Behälter, in die das Lebensmittel abgefüllt wird, oder Verschlüsse für die Behälter.

Im derzeitigen Stand der Technik erfolgt eine Desinfektion von Komponenten einer Abfüllanlage dadurch, dass der Betrieb der Abfüllanlage in regelmäßigen Abständen unterbrochen wird und die zu desinfizierenden Komponenten der Abfüllanlage mit einer recht hoch konzentrierten wässrigen Wasserstoffperoxidlösung beaufschlagt werden, die Keime, insbesondere Bakterien, abtötet. Bei einigen Anlagen erfolgt die Beaufschlagung mit der Wasserstoffperoxidlösung auch während des Betriebs. Um die Desinfektionswirkung zuverlässig zu erreichen wird typischerweise eine mindestens 30%ige Wasserstoffperoxidlösung zur Desinfektion verwendet. Da die Desinfektion in sehr regelmäßigen Abständen durchgeführt werden muss, hat dies den Nachteil, dass der Betreiber einer Abfüllanlage recht große Mengen von Wasserstoffperoxid bevorraten muss, um einen sicheren und vorschriftsmäßigen Betrieb der Abfüllanlage gewährleisten zu können. Zudem weisen Wasserstoffperoxidlösungen nur eine begrenzte Haltbarkeit auf, wodurch eine umfangreiche Lagerung erschwert wird.

Neben der Desinfektion von Komponenten einer Abfüllanlage werden wässrige Wasserstoffperoxidlösungen auch zur Desinfektion anderer Oberflächen eingesetzt, wie zum Beispiel von Fußböden in Krankenhäusern. Auch bei diesen Anwendungen ist eine recht hohe Wasserstoffperoxid-Konzentration von beispielsweise mindestens 30 Gew.-% erforderlich, so dass große Mengen an Wasserstoffperoxid bzw. bereits vorbereiteter Wasserstoffperoxidlösungen bevorratet werden müssen.

Alternativ kann die aus JP2001054556A, WO2017064741A1, WO2007071720A1 bekannte Plasmadesinfektion angewendet werden. Bei diesen Sterilisationsverfahren wird eine Reinigungsflüssigkeit in gasförmiger und/oder vernebelter Form mit dem Arbeitsgas einer Plasmaquelle vermischt oder in einen Plasmastrahl injiziert. Bei den in diesen Druckschriften beschriebenen Verfahren erfolgt die Plasmaeinwirkung erst nach dem Verdampfen oder Vernebeln der (noch nicht plasmaaktivierten) Flüssigkeit.

Vor diesem Hintergrund liegt der vorliegenden Erfindung die Aufgabe zugrunde, ein Verfahren zur Desinfektion von Oberflächen, insbesondere von Komponenten einer Abfüllanlage und/oder von beim Betrieb der Abfüllanlage verwendeten Materialien zur Verfügung zu stellen, mit dem die zuvor genannten Nachteile vermieden oder zumindest reduziert werden.

Entsprechend einem ersten Aspekt der vorliegenden Offenbarung wird diese Aufgabe, gemäß Anspruch 1, erfindungsgemäß durch ein Verfahren zur Desinfektion von Komponenten einer Abfüllanlage gelöst, bei dem die zu desinfizierenden Komponenten mit plasmaaktiviertem Wasser beaufschlagt werden, wobei das plasmaaktivierte Wasser zur Beaufschlagung auf die zu desinfizierenden Komponenten verdampft, versprüht und/oder zerstäubt wird.

Entsprechend dem ersten Aspekt wird diese Aufgabe weiterhin, gemäß Anspruch 3, bei einer Abfüllanlage, die zum Abfüllen eines Produkts in Behälter eingerichtet ist und Beaufschlagungsmittel zum Beaufschlagen von Komponenten der Abfüllanlage mit einer Reinigungsflüssigkeit aufweist, wobei die Beaufschlagungsmittel dazu eingerichtet sind, die Komponenten mit plasmaaktiviertem Wasser zu beaufschlagen, und wobei die Beaufschlagungsmittel ein oder mehrere Düsen, die dazu eingerichtet sind, plasmaaktiviertes Wasser auf die zu desinfizierenden Komponenten zu sprühen, und/oder ein oder mehrere Verdampfer, die dazu eingerichtet sind, plasmaaktiviertes Wasser im Bereich der zu desinfizierenden Komponenten zu verdampfen, und/oder ein oder mehrere Zerstäuber, die dazu eingerichtet sind, plasmaaktiviertes Wasser im Bereich der zu desinfizierenden Komponenten zu zerstäuben, umfassen, erfindungsgemäß dadurch gelöst, dass die Beaufschlagungsmittel mit einem Reservoir verbunden sind, das plasmaaktiviertes Wasser enthält, oder dass die Beaufschlagungsmittel mit einer Vorrichtung zur Herstellung von plasmaaktiviertem Wasser verbunden sind.

Unter plasmaaktiviertem Wasser wird Wasser verstanden, das durch die Einwirkung von einem aus einer atmosphärischen Plasmaquelle austretenden Arbeitsgas aktiviert worden ist. Insbesondere kann das Wasser unmittelbar mit atmosphärischem Plasma, wie zum Beispiel einem atmosphärischen Plasmastrahl, beaufschlagt werden, das heißt mit einem aus der Plasmaquelle austretenden Arbeitsgas, das sich zumindest teilweise noch im Plasmazustand befindet. Weiterhin kann das Wasser auch mit dem aus der Plasmaquelle austretenden Arbeitsgas beaufschlagt werden, nachdem das Arbeitsgas bereits wieder rekombiniert ist, das heißt sich nicht mehr im Plasmazustand befindet. Es wurde festgestellt, dass auch in einem solchen rekombinierten Arbeitsgas noch ausreichend reaktive Spezies, beispielsweise Ozon oder Stickoxide, enthalten sind, die im Wasser relativ langlebige reaktive Spezies wie zum Beispiel Wasserstoffperoxid, Salpetersäure oder salpetrige Säure bilden.

Entsprechend kann das plasmaaktivierte Wasser durch Einwirkung eines aus einer atmosphärischen Plasmaquelle austretenden Arbeitsgases auf (insbesondere flüssiges) Wasser hergestellt werden bzw. hergestellt sein.

Es wurde erkannt, dass sich das Wasser durch die Aktivierung mittels eines atmosphärischen Plasmas oder des rekombinierten Arbeitsgases mit reaktiven Spezies anreichern lässt, die sich über längere Zeit im Wasser halten und eine gute Desinfektionswirkung zeigen, wenn das plasmaaktivierte Wasser auf eine zu desinfizierenden Oberflächen aufgebracht wird.

Im Gegensatz zu konventionellen Lösungen mit Wasserstoffperoxid hat die Verwendung von plasmaaktiviertem Wasser den Vorteil, dass Wasserstoffperoxid nicht oder jedenfalls nur noch in kleineren Mengen bevorratet werden muss, da sich das Wasserstoffperoxid und gegebenenfalls andere reaktive Spezies durch die Plasmaaktivierung im Wasser bilden. Weiterhin wurde festgestellt, dass das plasmaaktivierte Wasser auch bei geringerer Wasserstoffperoxidkonzentration die gleiche Desinfektionswirkung zeigt wie eine herkömmliche Wasserstoffperoxidlösung ohne Plasmaaktivierung.

Für die Herstellung des plasmaaktivierten Wassers kann auch mit Wasserstoffperoxid versetztes Wasser verwendet werden, insbesondere eine wässrige Wasserstoffperoxid-Lösung mit einer Wasserstoffperoxid-Konzentration von vorzugsweise 1 bis 10 Gew.-%. Eine solche Wasserstoffperoxid-Lösung kann dann durch Einwirkung eines aus einer atmosphärischen Plasmaquelle austretenden Arbeitsgases plasmaaktiviert werden. Es hat sich herausgestellt, dass durch eine gewisse Wasserstoffperoxid-Konzentration im Wasser vor der Plasmaaktivierung eine noch bessere Sterilisierungswirkung des dadurch hergestellten plasmaaktivierten Wassers erreicht werden kann.

Das Verfahren dient zur Desinfektion von Komponenten einer Abfüllanlage. Bei den zu desinfizierenden Komponenten der Abfüllanlage kann es sich insbesondere um Teile der Abfüllanlage handeln, die mit dem abzufüllenden Produkt, insbesondere Lebensmittel, oder mit den beim Betrieb der Abfüllanlage verwendeten Materialien in Kontakt kommen.

Die Beaufschlagungsmittel der Abfüllanlage sind dazu eingerichtet, die Komponenten mit plasmaaktiviertem Wasser zu beaufschlagen. Zu diesem Zweck sind die Beaufschlagungsmittel mit einem Reservoir verbunden, das plasmaaktiviertes Wasser enthält, oder mit einer Vorrichtung zur Herstellung von plasmaaktiviertem Wasser. Dies hat den Vorteil, dass das plasmaaktivierte Wasser bedarfsgemäß hergestellt und direkt verwendet werden kann und eine Bevorratung entbehrlich ist.

Die Abfüllanlage ist dazu eingerichtet, ein Produkt, insbesondere ein Lebensmittel, in Behälter, insbesondere in gleichartige Behälter, abzufüllen. Zu diesem Zweck umfasst die Abfüllanlage vorzugsweise eine Abfülleinrichtung mit einer Abfülldüse, aus der im Betrieb das abzufüllende Produkt austritt, und mit einer Behälterführung, um einen Behälter unterhalb der Abfülldüse zu positionieren.

Weiterhin weist die Abfüllanlage vorzugsweise Transportmittel auf, die dazu eingerichtet sind, Behälter nacheinander durch die Abfüllanlage zu transportieren. Beispielsweise können die Transportmittel ein Abfüll-Karussell umfassen, mit dem die Behälter zum Befüllen auf einer Kreisbahn unter mitbewegten Abfülldüsen verfahren werden.

Die Abfüllanlage kann weiterhin eine der Abfülleinrichtung vorgelagerte Reinigungseinrichtung zum Reinigen der Behälter vor dem Befüllen aufweisen. Beispielsweise können die Transportmittel die Behälter zunächst durch eine Reinigungsstrecke fahren, in der die Behälter gereinigt werden, bevor diese zu der bzw. durch die Abfüllanlage weitertransportiert werden.

Die Reinigungsstrecke kann beispielsweise eine Beaufschlagungseinrichtung aufweisen, um die Behälter mit einer Reinigungsflüssigkeit zu beaufschlagen, sowie optional eine der Beaufschlagungseinrichtung nachgelagerte Abspüleinrichtung, um Reinigungsflüssigkeitsreste von den Behältern abzuspülen.

Die Beaufschlagungseinrichtung kann insbesondere dazu eingerichtet sein, die Behälter mit plasmaaktiviertem Wasser zu beaufschlagen. Beispielsweise können Düsen vorgesehen sein, die die Außenseiten der Behälter mit plasmaaktiviertem Wasser beaufschlagen.

Weiterhin können auch Düsen vorgesehen sein, um die Innenseiten der Behälter mit plasmaaktiviertem Wasser zu beaufschlagen. Alternativ können auch Plasmaquellen vorgesehen sein, die dazu eingerichtet sind, die Innenseite der Behälter mit einem atmosphärischen Plasma zu beaufschlagen.

Die Abfüllanlage kann weiterhin eine Verschließeinrichtung zum Verschließen der Behälter aufweisen, beispielsweise mit separaten Verschlüssen oder auch durch Verschweißen oder Verkleben des Behälters. Vorzugsweise kann die Abfüllanlage auch eine Reinigungsvorrichtung zum Reinigen von Verschlüssen aufweisen, die zum Verschließen der Behälter verwendet werden. Insbesondere kann die Reinigungseinrichtung Beaufschlagungsmittel aufweisen, die die Verschlüsse mit plasmaaktiviertem Wasser beaufschlagen.

Für das zuvor beschriebene Verfahren und die zuvor beschriebene Abfüllanlage ist plasmaaktiviertes Wasser erforderlich. Vor diesem Hintergrund kann eine Vorrichtung zur Herstellung von plasmaaktiviertem Wasser vorgesehen sein mit einem Aktivierungsraum zur Aufnahme eines Wasservolumens, mit einem Tellerbelüfter, der eine gasdurchlässige Membran aufweist, die an den Aktivierungsraum angrenzt, und mit einer Plasmaquelle zur Erzeugung eines atmosphärischen Plasmas in einem Arbeitsgas, wobei die Plasmaquelle eine Düsenöffnung aufweist, aus der im Betrieb das Arbeitsgas austritt, wobei die Plasmaquelle derart an den Tellerbelüfter angeschlossen ist, dass das im Betrieb aus der Plasmaquelle austretende Arbeitsgas durch die Membran des Tellerbelüfters in den Aktivierungsraum gelangt.

Das plasmaaktivierte Wasser kann mit einem Verfahren zur Herstellung von plasmaaktiviertem Wasser herstellt werden oder sein, insbesondere unter Verwendung der zuvor beschriebenen Vorrichtung, bei dem in einem Entladungsraum einer Plasmaquelle ein atmosphärisches Plasma in einem Arbeitsgas erzeugt wird, vorzugsweise mittels dielektrisch behinderter Entladung, und bei dem das Arbeitsgas aus dem Entladungsraum über einen Tellerbelüfter in ein Wasservolumen eingeleitet wird.

Es wurde festgestellt, dass sich das Arbeitsgas über einen Tellerbelüfter besonders gut in das Wasser einbringen lässt, um im Wasser reaktive Spezies anzureichern und dadurch plasmaaktiviertes Wasser herzustellen.

Die Vorrichtung zur Herstellung von plasmaaktiviertem Wasser weist einen Aktivierungsraum zur Aufnahme eines Wasservolumens sowie einen Tellerbelüfter auf, der eine gasdurchlässige Membran aufweist, die an den Aktivierungsraum grenzt. Der Aktvierungsraum kann beispielsweise durch einen Behälter, wie zum Beispiel einen Glaszylinder, gebildet werden, an dessen Unterseite ein Tellerbelüfter vorgesehen ist, sodass die gasdurchlässige Membran den Boden des Aktivierungsraums bildet. Bei der gasdurchlässigen Membran kann es sich beispielsweise um eine Membran mit einer Vielzahl, vorzugsweise tausender kleiner Öffnungen handeln, durch die das Gas in Form kleiner Bläschen in das im Aktivierungsraum befindliche Wasservolumen eingeleitet wird. Die Membran kann zum Beispiel aus einem Kunststoff oder Gummi, beispielsweise aus Ethylen-Propylen-Dien-Kautschuk (EPDM) bestehen.

Die Vorrichtung zur Herstellung von plasmaaktiviertem Wasser weist weiterhin eine Plasmaquelle zur Erzeugung eines atmosphärischen Plasmas in einem Arbeitsgas auf. Die Plasmaquelle kann insbesondere zur Erzeugung des Plasmas mittels dielektrisch behinderter Entladungen eingerichtet sein. Zu diesem Zweck weist die Plasmaquelle vorzugsweise zwei Elektroden mit einem dazwischen angeordneten Entladungsraum auf, wobei mindestens eine der Elektroden durch ein Dielektrikum, beispielsweise eine Keramik, vom Entladungsraum isoliert ist. Weiterhin ist vorzugsweise eine Spannungsversorgung vorgesehen, die zur Beaufschlagung der Elektroden mit einer hochfrequenten Hochspannung eingerichtet ist. Im Betrieb werden die beiden Elektroden über die Spannungsversorgung mit einer hochfrequenten Hochspannung beaufschlagt. Da das Dielektrikum direkte Entladungen zwischen den Elektroden verhindert, kommt es im Entladungsraum zu sogenannten dielektrisch behinderten Entladungen, die auch als dielektrische Barriereentladungen bezeichnet werden.

Die hochfrequente Hochspannung zum Betrieb der Plasmaquelle weist vorzugsweise eine Spannungsamplitude im Bereich von 1 kV bis 40 kV, vorzugsweise von 5 kV bis 20 kV, bei einer Frequenz im Bereich von 10 kHz bis 40 kHz, vorzugsweise 12 kHz bis 18 kHz, auf. Es wurde festgestellt, dass beim Betrieb der Plasmaquelle bei einer Frequenz im Bereich von 12 kHz bis 18 kHz besonders viel Ozon erzeugt wird, wodurch eine besonders gute Desinfektionswirkung des plasmaaktivierten Wassers erreicht werden konnte.

Unter der hochfrequenten Hochspannung zum Betrieb der Plasmaquelle wird die Spannung verstanden, die im Betrieb zwischen den Elektroden anliegt. Bei Einsatz eines Transformators zum Betrieb der Plasmaquelle handelt es sich demnach beispielsweise um die sekundärseitige Spannung.

Die Plasmaquelle weist eine Düsenöffnung auf, aus der im Betrieb das Arbeitsgas austritt. Das Arbeitsgas, das die Plasmaquelle im Betrieb durchströmt, kommt im Entladungsraum mit den dort auftretenden dielektrisch behinderten Entladungen in Kontakt und wird dadurch zumindest zum Teil in den Plasmazustand überführt, so dass ein atmosphärisches Plasma erzeugt wird. In dem Arbeitsgas bilden sich durch die Plasmaerzeugung reaktive Spezies, wie zum Beispiel Ozon oder Stickoxide, die auch nach der Rekombination des Arbeitsgases zumindest teilweise im Arbeitsgas erhalten bleiben. Das aus der Düsenöffnung der Plasmaquelle austretende Arbeitsgas enthält daher reaktive Spezies, selbst wenn die im Entladungsraum ionisierten Bestandteile des Arbeitsgases bereits wieder rekombiniert sind, so dass sich das Arbeitsgas an der Düsenöffnung nicht mehr im Plasmazustand befindet.

Als Arbeitsgas wird vorzugsweise Luft verwendet. Neben der einfachen Verfügbarkeit von Luft wurde dadurch auch eine besonders gute Desinfektionswirkung des plasmaaktivierten Wassers erreicht.

Die Plasmaquelle ist bei der Vorrichtung zur Herstellung von plasmaaktiviertem Wasser derart an den Tellerbelüfter angeschlossen, dass das im Betrieb aus der Plasmaquelle austretende Arbeitsgas durch die Membran des Tellerbelüfters in den Aktivierungsraum gelangt. Zu diesem Zweck ist die Düsenöffnung der Plasmaquelle insbesondere durch eine Leitung direkt mit dem Tellerbelüfter verbunden.

Plasmaaktiviertes Wasser, insbesondere hergestellt mit der zuvor beschriebenen Vorrichtung und/oder mit dem zuvor beschriebenen Verfahren, kann insbesondere zur Desinfektion von Komponenten oder Materialien einer Abfüllablage verwendet werden.

Insbesondere hat sich herausgestellt, dass sich plasmaaktiviertes Wasser über einen längeren Zeitraum von mehreren Tagen oder sogar Wochen lagern lässt, ohne dass es seine desinfizierende Wirkung verliert. Die Verwendung des plasmaaktivierten Wassers kann dadurch zeitlich und/oder räumlich getrennt von der Herstellung des plasmaaktivierten Wassers erfolgen. Beispielsweise kann das plasmaaktivierte Wasser nach der Herstellung und vor der Verwendung einfach in flüssiger Form gelagert werden.

Im Folgenden werden verschiedene Ausführungsformen des Verfahrens zur Desinfektion und der Abfüllanlage beschrieben, wobei die einzelnen Ausführungsformen jeweils unabhängig voneinander für das Verfahren und die Abfüllanlage gelten und auch untereinander kombiniert werden können.

Bei der ersten Ausführungsform des Verfahrens zur Desinfektion wird das plasmaaktivierte Wasser mit dem zuvor beschriebenen Verfahren zur Herstellung von plasmaaktiviertem Wasser hergestellt. Bei einer weiteren Ausführungsform ist das plasmaaktivierte Wasser mit dem zuvor beschriebenen Verfahren zur Herstellung von plasmaaktiviertem Wasser hergestellt. Es wurde festgestellt, dass das mit dem zuvor beschriebenen Verfahren hergestellte plasmaaktivierte Wasser besonders gut zur Desinfektion von Komponenten einer Abfüllanlage und von beim Betrieb der Abfüllanlage verwendeten Materialien geeignet ist, da es eine hohe Konzentration von langlebigen Spezies aufweist, die eine zuverlässige Desinfektion bewirken und dadurch einen sicheren Betrieb der Abfüllanlage gewährleisten.

Bei einer weiteren Ausführungsform des Verfahrens zur Desinfektion wird das plasmaaktivierte Wasser zur Beaufschlagung auf die zu desinfizierenden Komponenten und/oder Materialien versprüht. Bei einer entsprechenden Ausführungsform der Abfüllanlage umfassen die Beaufschlagungsmittel ein oder mehrere Düsen, die dazu eingerichtet sind, plasmaaktiviertes Wasser auf die zu desinfizierenden Komponenten und/oder Materialien zu sprühen.

Bei einer weiteren Ausführungsform des Verfahrens zur Desinfektion wird das plasmaaktivierte Wasser zur Beaufschlagung auf die zu desinfizierenden Komponenten und/oder Materialien verdampft. Bei einer entsprechenden Ausführungsform der Abfüllanlage umfassen die Beaufschlagungsmittel ein oder mehrere Verdampfer, die dazu eingerichtet sind, plasmaaktiviertes Wasser im Bereich der zu desinfizierenden Komponenten und/oder Materialien zu verdampfen.

Bei einer weiteren Ausführungsform des Verfahrens zur Desinfektion wird das plasmaaktivierte Wasser zur Beaufschlagung auf die zu desinfizierenden Komponenten und/oder Materialien zerstäubt oder vernebelt. Bei einer entsprechenden Ausführungsform der Abfüllanlage umfassen die Beaufschlagungsmittel ein oder mehrere Zerstäuber, die dazu eingerichtet sind, plasmaaktiviertes Wasser im Bereich der zu desinfizierenden Komponenten und/oder Materialien zu zerstäuben oder zu vernebeln.

Das plasmaaktivierte Wasser liegt vor dem Beaufschlagen auf die zu desinfizierenden Komponenten und/oder Materialien also insbesondere in flüssiger Form vor. Auf diese Weise kann das plasmaaktivierte Wasser einfach gelagert werden, so dass sich die Herstellung des plasmaaktivierten Wassers und deren Verwendung für die Desinfektion zeitlich und/oder räumlich trennen lässt. Zur Beaufschlagung auf die zu desinfizierenden Komponenten und/oder Materialien kann das plasmaaktivierte Wasser dann entsprechend insbesondere verdampft, versprüht und/oder zerstäubt werden, um eine gleichmäßige Benetzung der zu desinfizierenden Oberflächen zu erreichen.

Durch das Verdampfen, Versprühen und/oder Zerstäuben bzw. Vernebeln des plasmaaktivierten Wassers lässt sich dies fein und großflächig auf die zu desinfizierenden Komponenten bzw. Materialien verteilen, sodass eine flächendeckende Desinfektion ermöglicht wird. Es wurde festgestellt, dass die Desinfektionswirkung des plasmaaktivierten Wassers auch nach dem Verdampfen, Versprühen und/oder Zerstäuben bzw. Vernebeln noch ausreichend groß ist.

Bei einer weiteren Ausführungsform der Abfüllanlage ist eine Vorrichtung zur Herstellung von plasmaaktiviertem Wasser vorgesehen, insbesondere die zuvor beschriebene Vorrichtung zur Herstellung von plasmaaktiviertem Wasser, und die Beaufschlagungsmittel sind derart an die Vorrichtung zur Herstellung von plasmaaktiviertem Wasser angeschlossen, dass von der Vorrichtung zur Herstellung von plasmaaktiviertem Wasser erzeugtes plasmaaktiviertes Wasser zu den Beaufschlagungsmitteln geleitet wird. Auf diese Weise lässt sich das für die Desinfektion benötigte plasmaaktivierte Wasser bedarfsgemäß herstellen und unmittelbar zur Desinfektion verwenden. Eine Lagerung von plasmaaktiviertem Wasser wird auf diese Weise entbehrlich.

Die vorgesehene Vorrichtung zur Herstellung von plasmaaktiviertem Wasser umfasst vorzugsweise einen Aktivierungsraum zur Aufnahme eines Wasservolumens, eine Plasmaquelle zur Erzeugung eines atmosphärischen Plasmas in einem Arbeitsgas, wobei die Plasmaquelle eine Düsenöffnung aufweist, aus der im Betrieb das Arbeitsgas austritt, und einen Belüfter, der dazu eingerichtet ist, das im Betrieb aus der Düsenöffnung austretende Arbeitsgas in den Aktivierungsraum zu leiten. Insbesondere ist der Belüfter dazu eingerichtet, ein in dem Aktivierungsraum vorhandenes Wasservolumen mit dem im Betrieb aus der Düsenöffnung austretenden Arbeitsgas zu belüften. Zu diesem Zweck weist der Belüfter vorzugsweise ein oder mehrere Auslassöffnungen im Bodenbereich des Aktivierungsraums auf, aus denen das Arbeitsgas in ein im Aktivierungsraum vorhandenes Wasservolumen gelangen kann. Bevorzugt ist der Belüfter als Tellerbelüfter ausgebildet, der eine gasdurchlässige Membran aufweist, die an den Aktivierungsraum grenzt, so dass das im Betrieb aus der Düsenöffnung austretende Arbeitsgas durch die Membran des Tellerbelüfters in den Aktivierungsraum gelangt.

Bei einer weiteren Ausführungsform weist die Vorrichtung zur Herstellung von plasmaaktiviertem Wasser einen Einlass zum Einleiten von Wasser in den Aktivierungsraum und einen von dem Einlass separaten Auslass auf, um Wasser aus dem Aktivierungsraum auszulassen. Dies erlaubt beispielsweise auch einen kontinuierlichen Betrieb der Vorrichtung, sodass beispielsweise kontinuierlich frisches Wasser durch den Einlass in den Aktivierungsraum eingeleitet und aktiviertes Wasser durch den Auslass aus dem Aktivierungsraum abgeführt und zum Beispiel der unmittelbaren Verwendung, beispielsweise den Beaufschlagungsmitteln einer Abfüllanlage, zugeführt werden kann.

Bei einer weiteren Ausführungsform ist die Plasmaquelle der Vorrichtung zur Herstellung von plasmaaktiviertem Wasser dazu eingerichtet, ein Plasma mittels Erzeugung elektrischer Entladungen in einem Arbeitsgas zu erzeugen, vorzugsweise mittels dielektrisch behinderter Entladungen zwischen zwei Elektroden. Es wurde festgestellt, dass die Herstellung eines atmosphärischen Plasmas mittels dielektrisch behinderter Entladung besonders geeignet für die Herstellung von plasmaaktiviertem Wasser ist. Derart hergestelltes plasmaaktiviertes Wasser zeigte in Versuchen eine stärkere antibakterielle Wirkung als plasmaaktiviertes Wasser, das mit einer anderen Art Plasmaquelle hergestellt wurde, beispielsweise einer Plasmaquelle, bei der das Plasma mittels hochfrequenter bogenartiger Entladungen in einem Arbeitsgas erzeugt wird.

Die oben genannte Aufgabe kann weiterhin gelöst werden durch ein Verfahren zur Desinfektion einer Oberfläche, bei dem die zu desinfizierende Oberfläche mit einer Reinigungsflüssigkeit beaufschlagt wird, bei dem in einer Plasmaquelle ein atmosphärisches Plasma in einem Arbeitsgas erzeugt wird, das aus einer Düsenöffnung der Plasmaquelle austritt, und bei dem die mit der Reinigungsflüssigkeit beaufschlagte Oberfläche mit dem aus der Plasmaquelle austretenden Arbeitsgas beaufschlagt wird.

Es wurde festgestellt, dass gegenüber einer reinen Beaufschlagung einer zu desinfizierenden Oberfläche mit einer Reinigungsflüssigkeit, beispielsweise einer wässrigen Wasserstoffperoxidlösung, durch die nachträgliche Beaufschlagung der mit Reinigungsflüssigkeit beaufschlagten Oberfläche mit einem aus einer Plasmadüse austretenden Arbeitsgas eine vergleichbare Desinfektionswirkung bereits bei geringeren Konzentrationen reaktiver Stoffe in der Reinigungsflüssigkeit erreicht werden kann. In Versuchen wurde beispielsweise mit einer 7%igen wässrigen Wasserstoffperoxidlösung durch das nachträgliche Beaufschlagen mit einem Plasmastrahl eine vergleichbare Desinfektionswirkung erreicht wie mit einer wässrigen 30%igen Wasserstoffperoxidlösung ohne entsprechende Beaufschlagung mit einem Plasmastrahl.

Bei dem Verfahren wird die mit der Reinigungsflüssigkeit beaufschlagte Oberfläche mit dem aus der Plasmaquelle austretenden Arbeitsgas beaufschlagt. Die Beaufschlagung mit dem Arbeitsgas erfolgt also dann, wenn die zu desinfizierende Oberfläche noch mit einem entsprechenden Wasserfilm der Reinigungsflüssigkeit bedeckt ist. Dies führt dazu, dass in der beaufschlagten Reinigungsflüssigkeit reaktive Spezies durch das Plasma erzeugt werden, die zu einer stärkeren Desinfektionswirkung der Reinigungsflüssigkeit führen und daher eine gute Desinfektion auch bei geringeren Konzentrationen von beispielsweise Wasserstoffperoxid in der ursprünglichen Reinigungsflüssigkeit zulassen.

Als Reinigungsflüssigkeit kann beispielsweise Wasser oder eine wasserhaltige Lösung, insbesondere eine wässrige Wasserstoffperoxidlösung, verwendet werden. Die Reinigungsflüssigkeit weist vorzugsweise eine Wasserstoffperoxidkonzentration im Bereich von 1-10 Gew.-% auf, insbesondere wurde festgestellt, dass die Wasserstoffperoxidkonzentration viel geringer sein kann als die zur Desinfektion üblicherweise verwendeten 30 Gew.-%.

Das zuvor beschriebene Verfahren kann vorzugsweise zur Desinfektion von Komponenten einer Abfüllanlage und/oder von beim Betrieb der Abfüllanlage verwendeten Materialien eingesetzt werden, wobei die zu desinfizierenden Komponenten und/oder die zu desinfizierenden Materialien mit der Reinigungsflüssigkeit beaufschlagt werden und wobei die mit der Reinigungsflüssigkeit beaufschlagten Komponenten und/oder Materialien mit dem aus der Plasmaquelle austretenden Arbeitsgas beaufschlagt werden.

Erfindungsgemäß wird die oben genannte Aufgabe entsprechend dem zweiten Aspekt gemäß Anspruch 6 durch ein Verfahren zur Desinfektion von Komponenten einer Abfüllanlage und/oder von beim Betrieb der Abfüllanlage verwendeten Materialien gelöst, bei dem die zu desinfizierenden Komponenten und/oder die zu desinfizierenden Materialien mit einer Reinigungsflüssigkeit beaufschlagt werden, bei dem in einer Plasmaquelle ein atmosphärisches Plasma in einem Arbeitsgas erzeugt wird, das aus einer Düsenöffnung der Plasmaquelle austritt, und bei dem die mit der Reinigungsflüssigkeit beaufschlagten Komponenten und/oder die mit der Reinigungsflüssigkeit beaufschlagten Materialien mit dem aus der Plasmaquelle austretenden Arbeitsgas beaufschlagt werden, wobei als Reinigungsflüssigkeit eine wässrige Wasserstoffperoxid-Lösung mit einer Wasserstoffperoxid-Konzentration im Bereich von 1 bis 10 Gew.-% verwendet wird.

Die Plasmaquelle ist vorzugsweise dazu eingerichtet, das atmosphärische Plasma mittels einer dielektrisch behinderten Entladung zu erzeugen. Es wurde festgestellt, dass ein auf diese Weise erzeugtes Plasma zu höheren Wasserstoffperoxidkonzentrationen in dem auf die zu desinfizierende Oberfläche aufgebrachten Wasserfilm aus Reinigungsflüssigkeit führt, sodass eine besonders gute Desinfektionswirkung erreicht werden kann. Insbesondere kann die Plasmaquelle wie die für den ersten Aspekt beschriebene Plasmaquelle ausgebildet sein und/oder betrieben werden.

Insbesondere kann die Plasmaquelle zwei Elektroden mit einem dazwischen angeordneten Entladungsraum aufweisen, wobei mindestens eine der Elektroden durch ein Dielektrikum, beispielsweise eine Keramik, vom Entladungsraum isoliert ist. Weiterhin ist vorzugsweise eine Spannungsversorgung vorgesehen, die zur Beaufschlagung der Elektroden mit einer hochfrequenten Hochspannung eingerichtet ist. Im Betrieb werden die beiden Elektroden über die Spannungsversorgung mit einer hochfrequenten Hochspannung beaufschlagt. Da das Dielektrikum direkte Entladungen zwischen den Elektroden verhindert, kommt es im Entladungsraum zu sogenannten dielektrisch behinderten Entladungen, die auch als dielektrische Barriereentladungen bezeichnet werden.

Die hochfrequente Hochspannung zum Betrieb der Plasmaquelle weist vorzugsweise eine Spannungsamplitude im Bereich von 1 kV bis 40 kV, vorzugsweise von 5 kV bis 20 kV, bei einer Frequenz im Bereich von 10 kHz bis 40 kHz, vorzugsweise 12 kHz bis 18 kHz, auf. Es wurde festgestellt, dass beim Betrieb der Plasmaquelle bei einer Frequenz im Bereich von 12 kHz bis 18 kHz besonders viel Ozon erzeugt wird, wodurch eine besonders gute Desinfektionswirkung erreicht werden konnte.

Unter der hochfrequenten Hochspannung zum Betrieb der Plasmaquelle wird die Spannung verstanden, die im Betrieb zwischen den Elektroden anliegt. Bei Einsatz eines Transformators zum Betrieb der Plasmaquelle handelt es sich demnach beispielsweise um die sekundärseitige Spannung.

Das Arbeitsgas, das die zuvor beschriebene Plasmaquelle im Betrieb durchströmt, kommt im Entladungsraum mit den dort auftretenden dielektrisch behinderten Entladungen in Kontakt und wird dadurch zumindest zum Teil in den Plasmazustand überführt, so dass ein atmosphärisches Plasma erzeugt wird. In dem Arbeitsgas bilden sich durch die Plasmaerzeugung reaktive Spezies, wie zum Beispiel Ozon oder Stickoxide, die auch nach der Rekombination des Arbeitsgases zumindest teilweise im Arbeitsgas erhalten bleiben. Das aus der Düsenöffnung der Plasmaquelle austretende Arbeitsgas enthält daher reaktive Spezies, selbst wenn die im Entladungsraum ionisierten Bestandteile des Arbeitsgases bereits wieder rekombiniert sind, so dass sich das Arbeitsgas an der Düsenöffnung nicht mehr im Plasmazustand befindet.

Als Arbeitsgas wird vorzugsweise Luft verwendet. Neben der einfachen Verfügbarkeit von Luft wurde dadurch auch eine besonders gute Desinfektionswirkung erreicht.

Die Abfüllanlage kann eine Einhausung aufweisen, die einen zumindest teilweise geschlossenen Raum definiert, in dem die zu desinfizierenden Komponenten und/oder Materialien angeordnet sind. Beispielsweise können die Abfülleinrichtung der Abfüllanlage und/oder eine ggf. vorgesehene Reinigungseinrichtung in einer Einhausung untergebracht sein. Die Beaufschlagungsmittel sind vorzugsweise dazu eingerichtet, die zu desinfizierenden Komponenten und/oder Materialien innerhalb der Einhausung mit der Reinigungsflüssigkeit zu beaufschlagen, und die Plasmaquelle ist vorzugsweise dazu eingerichtet, das im Betrieb aus der Düsenöffnung austretende Arbeitsgas in dem von der Einhausung definierten Raum zu verteilen. Insbesondere können auch mehrere Plasmaquellen vorgesehen sein, um aus den Plasmaquellen austretendes Arbeitsgas in dem von der Einhausung definierten Raum zu verteilen. Bei einer entsprechenden Ausführungsform des Verfahrens wird das Arbeitsgas aus der Düsenöffnung in einen zumindest teilweise geschlossenen Raum eingeleitet, in dem sich die zu desinfizierenden Komponenten und/oder Materialien befinden. Auf diese Weise kann eine Desinfektion eines gesamten Abschnitts einer Abfüllanlage erreicht werden, die sich innerhalb der Einhausung bzw. des durch diesen definierten Raums befindet.

Die zuvor für den ersten Aspekt der Offenbarung beschriebenen Merkmale können auch mit dem zweiten Aspekt der Offenbarung kombiniert werden. Entsprechend können die für den zweiten Aspekt der Offenbarung beschriebenen Merkmale auch mit dem ersten Aspekt der Offenbarung kombiniert werden.

Weitere Vorteile und Merkmale der vorliegenden Erfindung ergeben sich aus der nachfolgenden Beschreibung an Ausführungsbeispielen, wobei auf die beigefügte Zeichnung Bezug genommen wird.

In der Zeichnung zeigen
- Fig. 1: eine Plasmaquelle zur Erzeugung eines atmosphärischen Plasmas,
- Fig. 2: ein Ausführungsbeispiel der Vorrichtung zur Herstellung von plasmaaktiviertem Wasser,
- Fig. 3: ein Ausführungsbeispiel des Verfahrens entsprechend dem ersten Aspekt der vorliegenden Erfindung,
- Fig. 4: ein Ausführungsbeispiel des Verfahrens gemäß dem zweiten Aspekt der vorliegenden Erfindung,
- Fig. 5: ein Ausführungsbeispiel einer erfindungsgemäßen Abfüllanlage,
- Fig. 6: ein Ausschnitt der Abfüllanlage aus Fig. 5,
- Fig. 7: ein weiteres Ausführungsbeispiel einer erfindungsgemäßen Abfüllanlage,
- Fig. 8: eine weitere Plasmaquelle zur Erzeugung eines atmosphärischen Plasmas,
- Fig. 9: eine andere Ausgestaltung der Plasmaquelle aus Fig. 1,
- Fig. 10: ein Beispiel eines weiteren Verfahrens und
- Fig. 11: ein Diagramm mit Versuchsergebnissen.

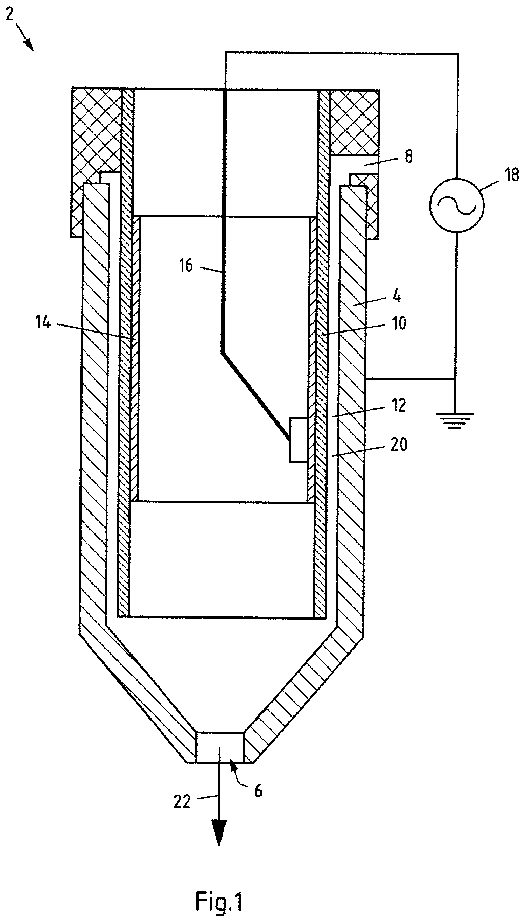

Figur 1 zeigt eine Plasmaquelle zur Erzeugung eines atmosphärischen Plasmas. Die Plasmaquelle 2 umfasst ein Düsenrohr 4 aus Metall, das sich zu einem Ende konisch zu einer Düsenöffnung 6 verjüngt. An dem der Düsenöffnung 6 gegenüberliegenden Ende des Düsenrohrs 4 ist ein Gaseinlass 8 für ein Arbeitsgas vorgesehen. In dem Düsenrohr 4 ist ein Keramikrohr 10 derart angeordnet, dass zwischen dem Keramikrohr 10 und dem Düsenrohr 4 ein ringförmiger Kanal 12 gebildet wird, durch den das Arbeitsgas vom Gaseinlass 8 zur Düsenöffnung 6 strömen kann.

Auf der Innenseite des Keramikrohrs 10 ist eine zylinderförmige Innenelektrode 14 angeordnet, die über ein Hochspannungskabel 16 mit einer Hochspannungsquelle 18 verbunden ist. Das Düsenrohr 4 ist geerdet und stellt dadurch eine Außenelektrode dar.

Im Betrieb wird durch den Gaseinlass 8 von einer nicht dargestellten Arbeitsgasquelle ein Arbeitsgas, vorzugsweise Luft, in die Plasmaquelle 2 eingeführt. Weiterhin wird mittels der Hochspannungsversorgung 18 eine hochfrequente Hochspannung zwischen der Innenelektrode 14 und dem als Außenelektrode wirkenden Düsenrohr 4 angelegt. Da das Keramikrohr 10 die Innenelektrode 14 gegenüber der Außenelektrode 4 elektrisch isoliert, können keine direkten Entladungsbögen zwischen der Innen- und der Außenelektrode auftreten. Stattdessen kommt es zu sogenannten dielektrisch behinderten Entladungen, bei der elektrische Entladungen in dem Entladungsraum 20 zwischen der Innen- und Außenelektrode auftreten.

Durch die Entladungen im Entladungsraum 20 wird das durch den Kanal 12 strömende Arbeitsgas zumindest teilweise in einen Plasmazustand überführt und damit ein atmosphärisches Plasma erzeugt. Das Arbeitsgas gelangt dann als Arbeitsgasstrom 22 durch die Düsenöffnung 6 aus der Plasmadüse 2 heraus.

Das aus der Plasmadüse 2 austretende Arbeitsgas 22 kann sich beim Austreten aus der Düsenöffnung 6 noch teilweise im Plasmazustand befinden. Es ist jedoch auch denkbar, dass das Arbeitsgas 22 bis zur Düsenöffnung 6 wieder vollständig rekombiniert ist, sodass sich das Arbeitsgas 22 nicht mehr im Plasmazustand befindet.

Durch die Anregung des Arbeitsgases in der Entladungszone 20 weist das aus der Plasmadüse 2 austretende Arbeitsgas jedoch reaktive Spezies, insbesondere Ozon und/oder Stickoxide, auf.

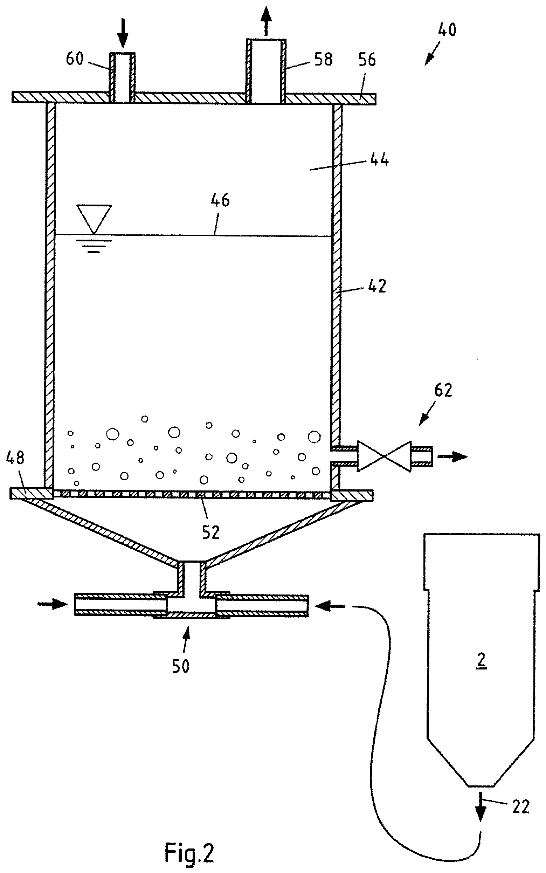

Figur 2 zeigt ein erstes Ausführungsbeispiel der Vorrichtung zur Herstellung von plasmaaktiviertem Wasser in schematischer Schnittansicht.

Die Vorrichtung 40 umfasst einen Behälter 42 in Form eines Glaszylinders, der einen Aktivierungsraum 44 zur Aufnahme eines Wasservolumens 46 umgibt. Anstelle eines Glaszylinders können auch andersartig geformte Behälter verwendet werden. Weiterhin kann der Behälter zum Beispiel auch aus Kunststoff, vorzugsweise PVC, oder Metall bestehen. Am Boden des Behälters ist ein Tellerbelüfter 48 vorgesehen, der eine Zuleitung 50 für ein Arbeitsgas aufweist sowie eine Membran 52, die den Boden des Aktivierungsraums 44 bildet und damit an den Aktivierungsraum 44 grenzt.

An die Zuleitung 50 ist die Plasmaquelle 2 aus Figur 1 derart angeschlossen, dass das aus der Plasmaquelle 2 austretende Arbeitsgas 22 über die Zuleitung 50 in den Tellerbelüfter 48 eingeleitet wird. Die gasdurchlässige Membran 52 weist eine Vielzahl, insbesondere tausender, kleiner Öffnungen (In der schematischen Fig. 2 sind die Öffnungen übertrieben groß dargestellt.) auf, durch die das Arbeitsgas aus der Plasmaquelle 2 in Form kleiner Bläschen in das Wasservolumen 46 gelangt. Auf diese Weise kommt das im Aktivierungsraum 44 befindliche Wasservolumen 46 in innige Berührung mit dem Arbeitsgasstrom 22 aus der Plasmaquelle 2, sodass die aktiven Spezies im Arbeitsgas 22, insbesondere Ozon und Stickoxide, langlebige reaktive Spezies, insbesondere Wasserstoffperoxid, salpetrige Säure oder Salpetersäure, in dem Wasservolumen 46 bilden. Auf diese Weise lässt sich das plasmaaktivierte Wasser herstellen.

Um den Anteil des Arbeitsgases, der nicht im Wasser 46 gelöst wird, kontrolliert absaugen zu können, weist der Behälter 42 einen Deckel 56 mit einem Absaugstutzen 58 auf, an dem eine nicht dargestellte Absaugeinrichtung angeschlossen ist. Weiterhin weist die Vorrichtung 40 einen Einlass 60 zum Einleiten von Wasser in den Aktivierungsraum 44 sowie einen davon separaten Auslass 62 zum Auslass des plasmaaktivierten Wassers aus dem Aktivierungsraum auf. Auf diese Weise ist auch ein kontinuierlicher Betrieb der Vorrichtung 40 möglich. Alternativ kann die Vorrichtung 40 auch batchweise betrieben werden.

Die Zuleitung 50 ist in Fig. 2 mit einem T-Stück ausgebildet, so dass zusätzlich zu dem Arbeitsgas 22 optional noch ein weiteres Gas in den Tellerbelüfter 48 geleitet werden kann, durch das sich die Konzentration der reaktiven Spezies im plasmaaktivierten Wasser beeinflussen lässt. Natürlich ist auch eine Ausgestaltung ohne T-Stück denkbar.

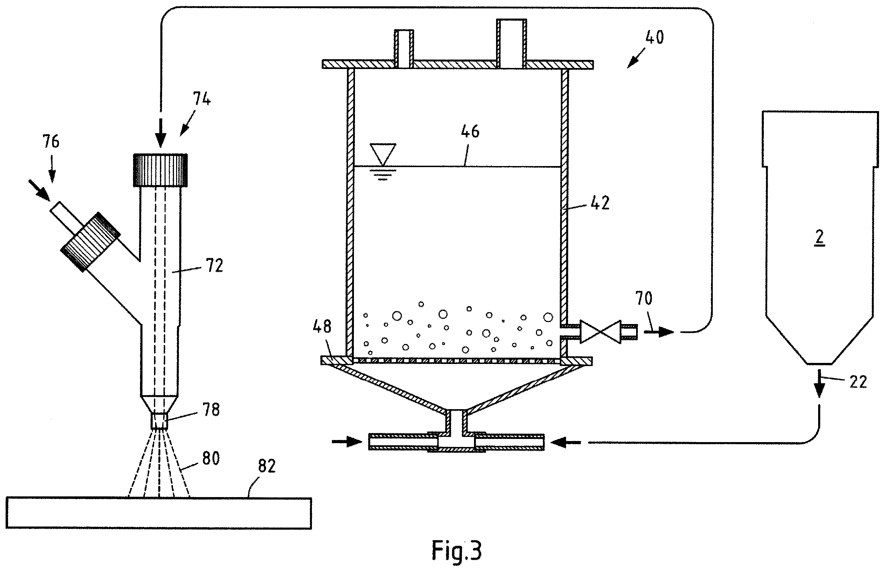

Figur 3 zeigt in schematischer Darstellung ein Ausführungsbeispiel des Verfahrens zur Desinfektion von Komponenten einer Abfüllanlage und/oder von beim Betrieb der Abfüllanlage verwendeten Materialien entsprechend dem ersten Aspekt.

Bei dem Verfahren wird plasmaaktiviertes Wasser 70 mit der in Figur 2 beschriebenen Vorrichtung hergestellt, indem mit der Plasmaquelle 2 ein atmosphärisches Plasma in einem Arbeitsgas erzeugt wird und das aus der Plasmaquelle austretende Arbeitsgas 22 durch den Tellerbelüfter 48 in das im Behälter 42 enthaltene Wasser 46 gelangt, so dass sich in dem Wasser 46 langlebige reaktive Spezies anreichern.

Das auf diese Weise hergestellte plasmaaktivierte Wasser 70 wird sodann von der Vorrichtung 40 zu einer Verneblungsdüse 72 geleitet und dort vernebelt. Zu diesem Zweck weist die Verneblungsdüse einen ersten Einlass 74 zur Zuführung des plasmaaktivierten Wassers 70 und einen zweiten Einlass 76 zur Zuführung eines Verneblungsgases auf. Am Düsenauslass 78 der Verneblungsdüse 72 vernebelt das Verneblungsgas das plasmaaktivierte Wasser 70, sodass ein Nebel 80 aus plasmaaktiviertem Wasser gebildet wird. Die Verneblungsdüse 72 ist auf die zu desinfizierende Komponente der Abfüllanlage bzw. beim Betrieb der Abfüllanlage verwendeten Materialien gerichtet, sodass deren Oberfläche 82 durch den Nebel 80 des plasmaaktivierten Wassers benetzt wird. Durch die reaktiven Spezies im plasmaaktivierten Wasser 70 wird dadurch eine Desinfektion der Oberfläche 82 erreicht.

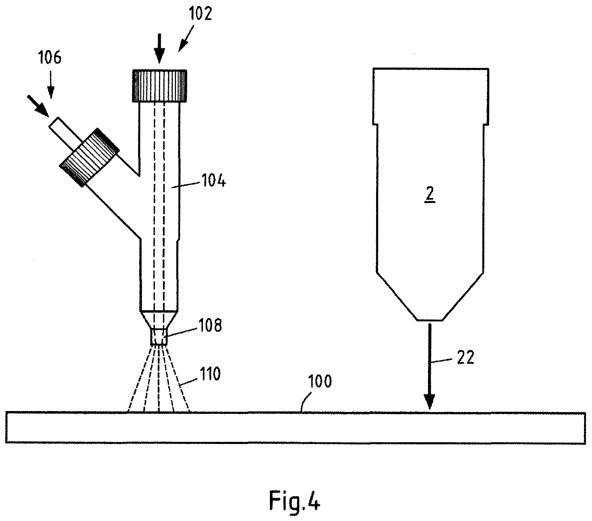

Figur 4 zeigt in schematischer Darstellung ein Ausführungsbeispiel des Verfahrens zur Desinfektion von Oberflächen entsprechend dem zweiten Aspekt.

Bei dem Verfahren wird eine zu desinfizierende Oberfläche 100 zuerst mit einer Reinigungsflüssigkeit 102, beispielsweise Wasser oder 3-7%iger wässriger Wasserstoffperoxidlösung, beaufschlagt, indem zum Beispiel die Reinigungsflüssigkeit 102 und ein Verneblungsgas 106, insbesondere Luft, in eine Verneblungsdüse 104 eingeleitet werden, so dass aus der Düsenöffnung 108 der Verneblungsdüse 104 ein Nebel 110 der Reinigungsflüssigkeit 102 austritt und die zu desinfizierende Oberfläche 100 benetzt.

Anschließend wird mit der Plasmaquelle 2 aus Fig. 1 ein atmosphärisches Plasma erzeugt und das aus der Düsenöffnung 6 der Plasmadüse austretende Arbeitsgas 22 wird auf die zuvor mit der Reinigungsflüssigkeit 102 benetzte zu desinfizierende Oberfläche 100 gerichtet. Durch die Wechselwirkung des Arbeitsgases 22 aus der Plasmaquelle 2 mit dem Reinigungsflüssigkeitsfilm auf der Oberfläche 100 kommt es zu einer verstärkten Desinfektionswirkung der Reinigungsflüssigkeit 102, sodass trotz einer recht geringen Konzentration von beispielsweise Wasserstoffperoxid in der Reinigungsflüssigkeit eine starke Desinfektionswirkung erreicht werden kann, für die ohne die anschließende Behandlung mit dem Arbeitsgas 22 eine viel höhere Wasserstoffperoxidkonzentration erforderlich wäre.

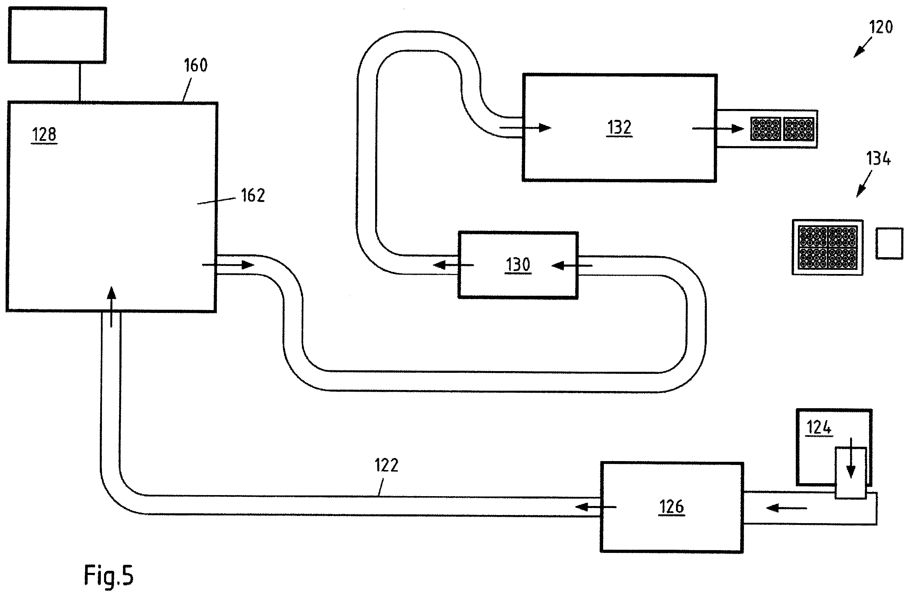

Die Figur 5 zeigt ein Ausführungsbeispiel einer erfindungsgemäßen Abfüllanlage in hoch schematischer Aufsicht. Bei der Abfüllanlage 120 handelt es sich um eine Getränkeabfüllanlage zum Abfüllen von Getränken in Flaschen. Die Abfüllanlage weist ein Transportsystem 122 auf, mit dem die Flaschen von einem Flaschenvorrat 124 durch die einzelnen Stationen der Abfüllanlage 120 transportiert werden. In einer ersten Station 126 werden die Flaschen ausgerichtet und vereinzelt, sodass sie in gleichmäßigen Abständen mit dem Transportsystem 120 durch die Anlage transportiert werden können.

In der zweiten Station 128 erfolgen eine innere und äußere Reinigung der Flaschen, das Befüllen mit dem Getränk und das Verschließen der Flaschen mit einem Deckel. In einer weiteren Station 130 werden die Flaschen dann noch etikettiert und in einer letzten Station 132 zu Gebinden verpackt, sodass diese auf Paletten 134 für den Vertrieb weitertransportiert werden können.

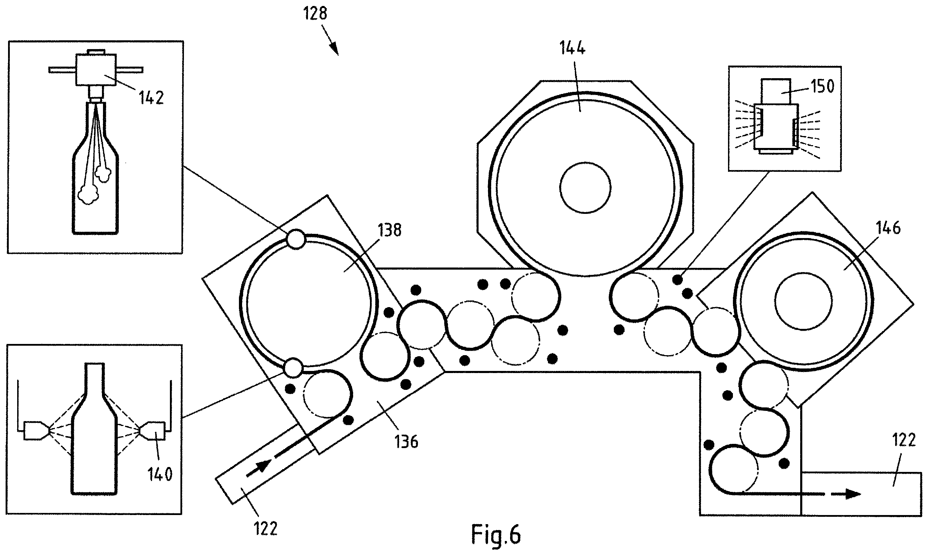

Figur 6 zeigt eine schematische Darstellung der Station 128 in Aufsicht. Die Flaschen werden von dem Transportsystem 122 zunächst zu einer Reinigungseinrichtung 136 geführt, in der die Flaschen von innen und außen gereinigt werden. Zu diesem Zweck werden die Flaschen an ein Reinigungs-Karussell 138 übergeben, in dem die Flaschen auf einer kreisförmigen Bahn transportiert werden, während sie mittels dafür vorgesehener Reinigungsmittel in Form von Düsen 140 und Injektordüsen 142 von außen und innen gereinigt werden.

Nach Durchlaufen des Reinigungs-Karussells 138 werden die Flaschen über Transportkreise an ein Abspül-Karussell 144 übergeben, in dem die Flaschen wiederum im Kreis geführt werden, während sie durch Beaufschlagung mit Wasser von Resten der Reinigungsflüssigkeit von der vorherigen Reinigung befreit werden. Anschließend werden die Flaschen zu einem Abfüll-Karussell 146 weitergeleitet, in dem die Flaschen wiederum im Kreis geführt und dabei durch mitlaufende Auslassdüsen mit einem Getränk befüllt und mit einem Deckel verschlossen werden.

Anschließend werden die Flaschen mit dem Transportsystem 122 zur nachfolgenden Station 130 weitertransportiert.

Die Station 128 muss aus Hygienegründen regelmäßig oder ggf. sogar permanent desinfiziert werden. Zu diesem Zweck sind in der Station verteilt Reinigungsdüsen 150 vorgesehen, mit denen die einzelnen Komponenten der Station 128 mit einer Reinigungsflüssigkeit beaufschlagt werden können.

Im Stand der Technik wurde zur Desinfektion der Anlage regelmäßig hochprozentige, beispielsweise 30%ige, wässrige Wasserstoffperoxidlösung verwendet. Dies erfordert jedoch eine Bevorratung großer Mengen an Wasserstoffperoxid.

Bei der in Figur 5 und 6 dargestellten Abfüllanlage sind die Düsen 150 jedoch an eine Vorrichtung zur Herstellung von plasmaaktiviertem Wasser angeschlossen, beispielsweise die Vorrichtung 40 aus Figur 2. Zur Desinfektion der Komponenten der Station 128 der Abfüllanlage 120 wird dann mit den Düsen 150 plasmaaktiviertes Wasser auf die einzelnen Komponenten gesprüht, wodurch eine gründliche Desinfektion der Komponenten erreicht werden kann, ohne dass große Menge Wasserstoffperoxid bevorratet werden müssen.

Weiterhin kann das plasmaaktivierte Wasser auch zur Reinigung der Flaschen verwendet werden, zum Beispiel indem das plasmaaktivierte Wasser über die Düsen 140 auf die Außenoberfläche der Flaschen gesprüht wird. Es ist auch denkbar, das Innere der Flaschen durch plasmaaktiviertes Wasser zu desinfizieren, indem das plasmaaktivierte Wasser über die Injektordüse 142 in die Flaschen hineingesprüht wird. Alternativ kann aus einer Plasmaquelle austretendes Arbeitsgas in die Flaschen eingeleitet werden.

Durch die Verwendung des plasmaaktivierten Wassers zur Desinfizierung von Komponenten der Abfüllanlage 120 bzw. beim Betrieb der Abfüllanlage 120 verwendeter Materialien in Form von Flaschen kann eine effektive Desinfizierung und ein sicherer Betrieb der Abfüllanlage 120 gewährleistet werden.

Bei einer alternativen Ausführung der Abfüllanlage entsprechend dem zweiten Aspekt können die Komponenten der Abfüllanlage 120 über die Düsen 150 auch zunächst mit einer Reinigungsflüssigkeit, beispielsweise Wasser oder einer zum Beispiel 3%igen wässrigen Wasserstoffperoxidlösung, und anschließend mit einem aus einer Plasmaquelle austretenden Arbeitsgas beaufschlagt werden. Zu diesem Zweck sind vorzugsweise eine Einhausung 160 um die Station 128 sowie mehrere Plasmadüsen vorgesehen, mit denen sich aus den Plasmadüsen austretendes Arbeitsgas im Innenraum 162 der Einhausung verteilen lässt (vgl. Fig. 5). Durch die Wechselwirkung des Reinigungsflüssigkeitsfilms auf den zu desinfizierenden Komponenten mit dem Arbeitsgas aus den Plasmadüsen wird die Desinfektionswirkung der Reinigungsflüssigkeit verstärkt, so dass die gewünschte Desinfektionswirkung bereits bei geringeren Wasserstoffperoxidkonzentrationen in der Reinigungsflüssigkeit erreicht werden kann.

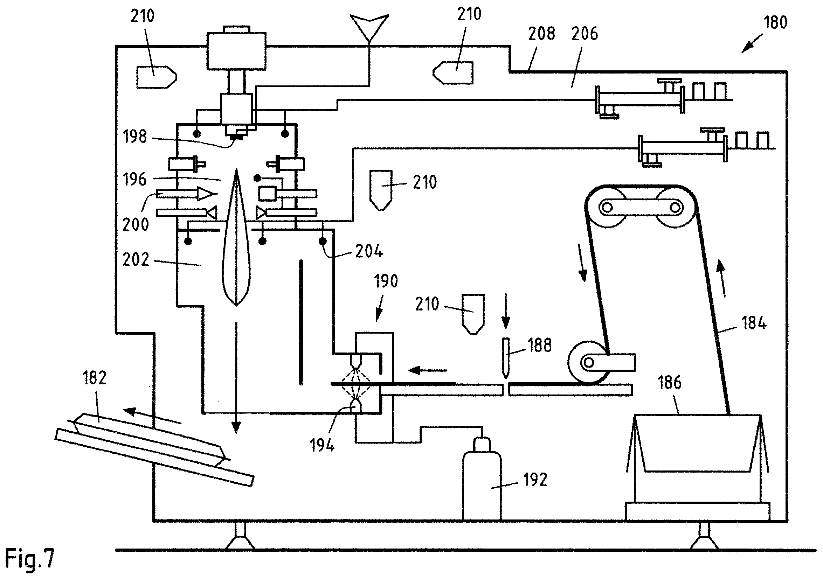

Figur 7 zeigt ein weiteres Ausführungsbeispiel einer Abfüllanlage 180. Die Abfüllanlage 180 ist als Getränkeabfüllanlage ausgebildet, um Milch in Schläuche 182 abzufüllen.

Beim Betrieb der Abfüllanlage 180 wird ein kontinuierlicher Schlauch 184 aus einem Schlauchvorrat 186 entnommen und an einer Schneideinrichtung 188 in Schlauchabschnitte vorgegebener Länge geschnitten. Die Schlauchabschnitte werden dann einer Reinigungseinrichtung 190 zugeführt, in der eine Reinigungsflüssigkeit aus einem Reservoir 192 über Düsen 194 auf die Außenseite der Schlauchabschnitte gesprüht wird. Die Schlauchabschnitte werden dann zu einer Füllstation 196 geführt, mittels eines Ultraschallschweißers 200 einseitig zugeschweißt, über eine vorgesehene Auslassdüse 198 mit Milch befüllt und anschließend mit dem Ultraschallschweißer 200 vollständig verschweißt. Anschließend werden die Schläuche durch einen sterilen Bereich 202 aus der Abfüllanlage 180 herausgeführt.

Bei einem Ausführungsbeispiel entsprechend dem ersten Aspekt wird als Reinigungsflüssigkeit zur Reinigung der Schlauchabschnitte in der Reinigungseinrichtung 190 plasmaaktiviertes Wasser verwendet, das in dem Reservoir 192 gelagert wird. Alternativ kann anstelle des Reservoirs 192 zum Beispiel auch die Vorrichtung 40 zur Herstellung von plasmaaktiviertem Wasser aus Figur 1 angeschlossen sein.

Zur Desinfektion der einzelnen Komponenten der Abfüllanlage 180 selbst können in der Anlage Düsen 204 vorgesehen sein, die plasmaaktiviertes Wasser in bestimmten Intervallen oder kontinuierlich auf die Komponenten der Anlage sprühen und diese dadurch desinfizieren.

Bei einem alternativen Ausführungsbeispiel entsprechend dem zweiten Aspekt werden die Komponenten der Abfüllanlage 180 über die Düsen 204 mit einer Reinigungsflüssigkeit, beispielsweise einer 3%igen wässrigen Wassersttoffperoxidlösung, beaufschlagt. Anschließend wird in den Innenraum 206 des die Abfüllanlage 180 umgebenden Gehäuses 208 über mehrere vorgesehene Plasmadüsen 210 ein Plasma bzw. ein aus den Plasmadüsen 210 austretendes Arbeitsgas eingeleitet und der Innenraum 206 damit geflutet, sodass die zuvor mit der Reinigungsflüssigkeit beaufschlagten Komponenten der Abfüllanlage 180 intensiver desinfiziert werden.

Wie die Beispiele aus den Figuren 6 und 7 zeigen, kann die erfindungsgemäße Lehre auf verschiedene Arten von Abfüllanlagen angewendet werden. Insbesondere kann plasmaaktiviertes Wasser zur Desinfektion verschiedenartiger Abfüllanlagen verwendet werden.

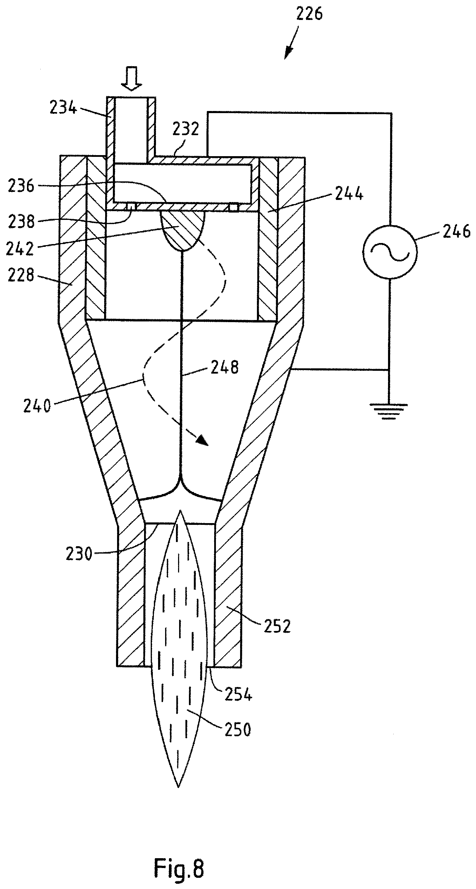

Fig. 8 zeigt in schematischer Schnittansicht eine weitere Plasmaquelle 226 zur Erzeugung eines atmosphärischen Plasmas in Form einer Plasmadüse. Die Plasmadüse 226 weist ein Düsenrohr 228 aus Metall auf, das sich im Wesentlichen konisch zu einer Düsenrohrmündung 230 verjüngt. An dem der Düsenrohrmündung 230 entgegen gesetzten Ende weist das Düsenrohr 228 eine Dralleinrichtung 232 mit einem Einlass 234 für ein Arbeitsgas, beispielsweise Luft, auf.

Eine Zwischenwand 236 der Dralleinrichtung 232 weist einen Kranz von schräg in Umfangsrichtung angestellten Bohrungen 238 auf, durch die das Arbeitsgas verdrallt wird. Der stromabwärtige, konisch verjüngte Teil des Düsenrohres 228 wird deshalb von dem Arbeitsgas in Form eines Wirbels 240 durchströmt, dessen Kern auf der Längsachse des Düsenrohrs 228 verläuft. An der Unterseite der Zwischenwand 36 ist mittig eine Elektrode 242 angeordnet, die koaxial in Richtung des verjüngten Abschnittes in das Düsenrohr 228 hineinragt. Die Elektrode 242 ist elektrisch mit der Zwischenwand 236 und den übrigen Teilen der Dralleinrichtung 232 verbunden. Die Dralleinrichtung 232 ist durch ein Keramikrohr 244 elektrisch gegen das Düsenrohr 228 isoliert. Über die Dralleinrichtung 232 wird an die Elektrode 242 eine hochfrequente Hochspannung angelegt, die von einem Transformator 246 erzeugt wird. Der Einlass 234 ist über einen nichtgezeigten Schlauch mit einer unter Druck stehenden Arbeitsgasquelle mit variablem Durchsatz verbunden. Das Düsenrohr 228 ist geerdet. Durch die angeregte Spannung wird eine Hochfrequenzentladung in Form eines Lichtbogens 248 zwischen der Elektrode 242 und dem Düsenrohr 228 erzeugt.

Die Begriffe "Lichtbogen", "Bogenentladung" bzw. "bogenartige Entladung" werden vorliegend als phänomenologische Beschreibungen der Entladung verwendet, da die Entladung in Form eines Lichtbogens auftritt. Der Begriff "Lichtbogen" wird anderweitig auch als Entladungsform bei Gleichspannungsentladungen mit im Wesentlichen konstanten Spannungswerten verwendet. Vorliegend handelt es sich jedoch um eine Hochfrequenzentladung in Form eines Lichtbogens, also um eine hochfrequente bogenartige Entladung.

Aufgrund der drallförmigen Strömung des Arbeitsgases wird dieser Lichtbogen 248 im Wirbelkern auf der Achse des Düsenrohres 228 kanalisiert, so dass er sich erst im Bereich der Düsenrohrmündung 230 zur Wand des Düsenrohrs 228 verzweigt.

Das Arbeitsgas, das im Bereich des Wirbelkerns und damit in unmittelbarer Nähe des Lichtbogens 248 mit hoher Strömungsgeschwindigkeit rotiert, kommt mit dem Lichtbogen 248 in innige Berührung und wird dadurch zum Teil in den Plasmazustand überführt, so dass ein atmosphärischer Plasmastrahl 250 durch die Düsenrohrmündung 230 in eine an die Düsenrohrmündung angrenzende Auslassdüse 252 gelangt.

Aus der Düsenöffnung 254 der Auslassdüse 252 tritt das Arbeitsgas dann in Form des Plasmastrahls 250 aus der Plasmadüse 226 heraus.

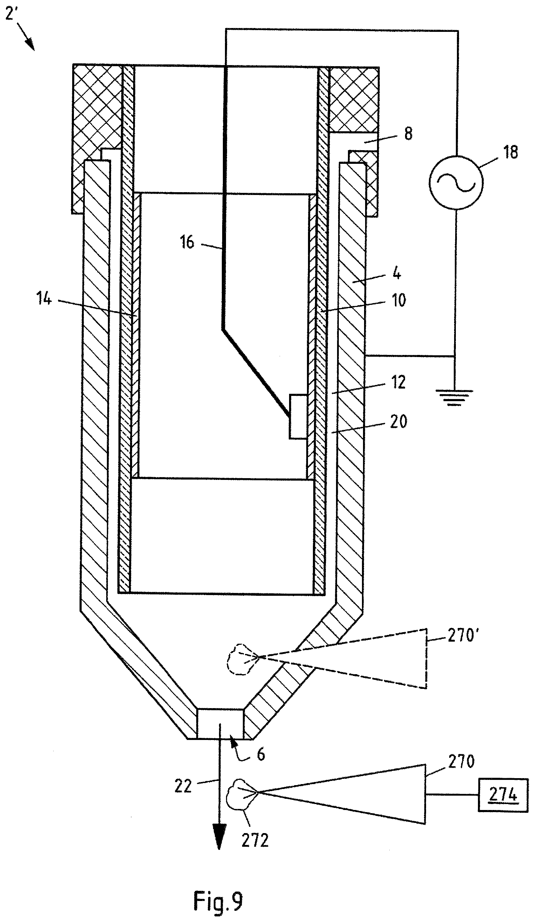

Fig. 9 zeigt eine andere Ausgestaltung der Plasmaquelle aus Fig. 1. Der Aufbau der Plasmaquelle 2' entspricht im Wesentlichen dem Aufbau der Plasmaquelle 2 aus Fig. 1. Gleiche Komponenten sind mit gleichen Bezugszeichen versehen. Zusätzlich weist die Plasmaquelle 2' eine Vorrichtung 270 auf, mit dem eine Reinigungsflüssigkeit in das aus der Plasmaquelle 2' austretende Arbeitsgas 22 eingeleitet werden kann. Bei der Vorrichtung 270 kann es sich beispielsweise um einen Verdampfer handeln, der dazu eingerichtet ist, eine Reinigungsflüssigkeit 272 zu verdampfen und in das Arbeitsgas 22 einzubringen. Alternativ kann es sich bei der Vorrichtung 270 auch um einen Zerstäuber bzw. Vernebler handeln, der dazu eingerichtet ist, eine Reinigungsflüssigkeit 272 zu zerstäuben bzw. zu vernebeln und in das Arbeitsgas 22 einzubringen. Weiterhin kann die Vorrichtung 270 auch dazu eingerichtet sein, eine Reinigungsflüssigkeit 272 in das Arbeitsgas 22 einzusprühen.

Bei der Reinigungsflüssigkeit 272 kann es sich insbesondere um Wasser, um eine wässrige Wasserstoffperoxidlösung oder um plasmaaktiviertes Wasser handeln.

Die Vorrichtung 270 ist mit einer Quelle 274 für die Reinigungsflüssigkeit verbunden. Bei der Quelle 274 kann es sich insbesondere um ein die Reinigungsflüssigkeit 272 enthaltendes Reservoir oder um eine Vorrichtung zur Herstellung von plasmaaktiviertem Wasser, wie zum Beispiel um die Vorrichtung 40 aus Fig. 2, handeln.

Die Vorrichtung 270 kann wie in Fig. 9 dargestellt im Bereich der Düsenöffnung 6 außerhalb des Düsenrohrs 4 angeordnet sein. Alternativ kann die Vorrichtung 270 auch wie in Fig. 9 gestrichelt dargestellt (Bezugszeichen 270') in das Düsenrohr 4 oder auch in eine am Düsenrohr 4 vorgesehene Düsenanordnung (nicht dargestellt) integriert werden.

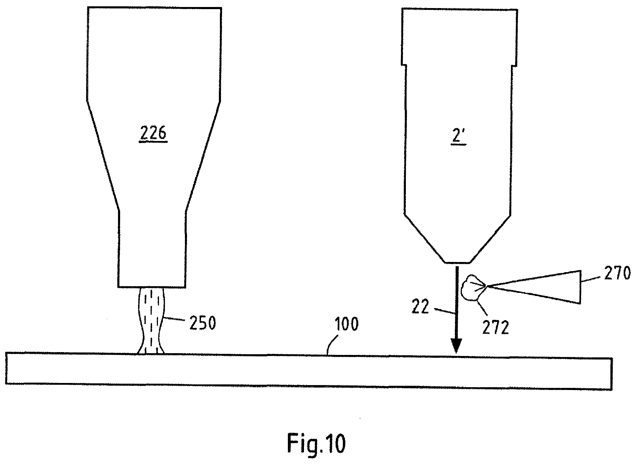

Fig. 10 zeigt in schematischer Darstellung ein Beispiel eines Verfahrens zur Desinfektion von Oberflächen.

Bei dem Verfahren wird die zu desinfizierende Oberfläche 100 zuerst mit dem aus der Plasmadüse 226 aus Fig. 8 austretenden atmosphärischen Plasmastrahl 250 beaufschlagt. Anschließend wird die auf diese Weise behandelte Oberfläche 100 mit dem Arbeitsgas 22 aus der Plasmadüse 2' aus Fig. 9 beaufschlagt, wobei mittels der im Bereich der Düsenöffnung 6 angeordneten Vorrichtung 270 eine Reinigungsflüssigkeit 272 zum Beispiel verdampft, vernebelt, zerstäubt oder versprüht und in das Arbeitsgas 22 eingebracht wird, so dass die Reinigungsflüssigkeit 272 und das Arbeitsgas 22 zusammen auf die Oberfläche 100 gelangen.

Bei der Oberfläche 100 kann es sich beispielsweise um die Oberfläche einer Komponente einer Abfüllanlage handeln, beispielsweise der Abfüllanlage 120 oder 180, oder von beim Betrieb der Abfüllanlage verwendeten Materialien.

Es hat sich gezeigt, dass sich Oberflächen mit dem in Fig. 10 dargestellten Verfahren effektiver und bei höheren Verfahrgeschwindigkeiten der Plasmaquellen 226 und 2' desinfiziert werden können.

Im Rahmen der Erfindung wurden Versuchsreihen mit einer Vorrichtung wie in Fig. 2 dargestellt durchgeführt, um die Anreicherung reaktiver Spezies in Wasser durch die Behandlung mit Arbeitsgas aus einer Plasmaquelle und damit die Herstellbarkeit von plasmaaktiviertem Wasser zu belegen.

Für die einzelnen Versuche der Versuchsreihen wurden jeweils ein Testvolumen von 2 l entweder einer 0,9%igen wässrigen NaCl-Lösung oder einer phosphorgepufferten wässrigen Salzlösung (PBS) in das Gefäß 42 gegeben und mit dem Tellerbelüfter 48 über verschiedene Zeitdauern mit dem Arbeitsgas aus der Plasmaquelle 2 beaufschlagt. Als Arbeitsgas wurde Luft verwendet.

Vor der Beaufschlagung mit dem Arbeitsgas wurden die Testvolumina jeweils mit Bakterien vom Typ E. coli versetzt. Vor Beginn und nach Ende der Beaufschlagung des Wassers mit dem Arbeitsgas 22 aus der Plasmaquelle 2 wurden jeweils Proben des Wassers entnommen und in mehreren Verdünnungsstufen auf je ein Viertel einer LB-Agar-Platte getropft. Die Proben wurden für 3 Tage bei 30°C inkubiert. Nach den drei Tagen wurden die Kolonien ausgezählt.

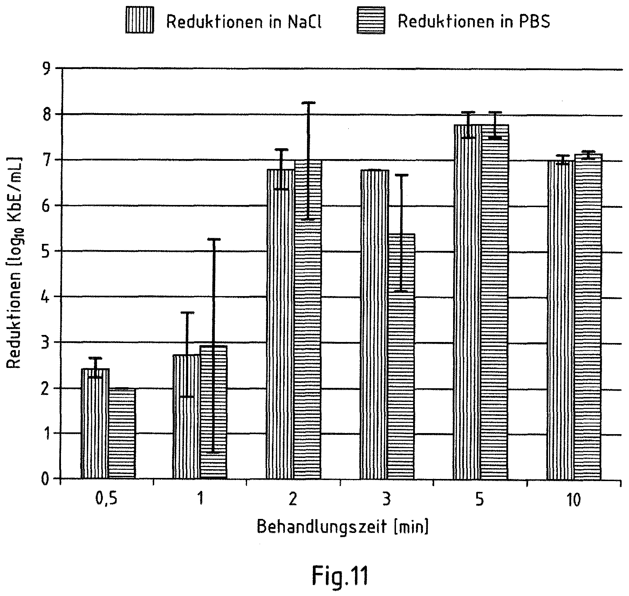

Die Ergebnisse der Versuche sind in Fig. 11 in Form eines Diagramms dargestellt:
In dem Diagramm in Fig. 11 zeigen die Balken die Reduktion der koloniebildenden Einheiten (KbE) pro Milliliter in Log10-Stufen, und zwar jeweils für die NaCl-Lösung (jeweils linker Balken) und für die PBS-Lösung (jeweils rechter Balken). Wie das Diagramm zeigt, konnte bereits nach einer Behandlungzeit des Wassers von 2 min. eine signifikante Reduktion der koloniebildenden Einheiten um ca. sieben Log10-Stufen erreicht werden. Dies bestätigt, dass die Beaufschlagung des Wassers mit dem Arbeitsgas 22 aus der Plasmaquelle 2 zu einer Anreicherung von reaktiven Spezies im Wasser führt, die eine stark desinfizierende Wirkung haben. Damit ist das auf diese Weise hergestellte plasmaaktivierte Wasser besonders gut zur Desinfektion geeignet.

Weiterhin wurden Versuche durchgeführt, die die Wirkung des Verfahrens entsprechend dem zweiten Aspekt belegen.

Hierzu wurden 21 handelsübliche und gleichartige Getränkeflaschen-Drehverschlüsse aus Polyethylen (PE) durch Auftropfen von jeweils 20 µl einer Testkeim-Lösung (Bacillus atrophaeus Sporen 9372) kontaminiert. Die Deckel wurden nach der Kontamination für 30 min. bei 30 °C getrocknet.

Von den 21 kontaminierten Drehverschlüssen wurden drei als Vergleichsproben nicht behandelt. Die anderen 18 kontaminierten Drehverschlüsse wurden mittels eines Zerstäubers mit demineralisiertem Wasser (VE-Wasser) oder einer wässrigen Wasserstoffperoxidlösung (3 Gew.-%, 7 Gew.-% oder 10 Gew.-%) besprüht. Ein Teil der besprühten Drehverschlüsse wurde zusätzlich für 30s mit Arbeitsgas aus einer Plasmaquelle beaufschlagt. Hierzu wurde eine Plasmadüse mit dem in Fig. 1 gezeigten Aufbau verwendet.

Nach der Behandlung wurden alle Deckel für je 5 min. inkubiert und anschließend mit je 1ml 0,9%iger NaCl-Lösung gespült. Für jede Probe wurde mit der aus dieser Spülung jeweils gewonnenen Abwaschlösung je eine logarithmische Verdünnungsreihe mit acht Verdünnungsstufen (10⁻¹ bis 10⁻⁸) hergestellt. Von jeder der acht Verdünnungsstufen je Probe wurden je 100µl auf je ein Viertel einer LB-Agar-Platte getropft. Die Proben wurden für 3 Tage bei 30°C inkubiert. Nach den drei Tagen wurden die Kolonien ausgezählt.

Die nachfolgende Tabelle 1 zeigt die Ergebnisse dieser Versuchsreihe. In der ersten Spalte ist jeweils die Behandlung der Deckel zu den jeweiligen Proben angegeben. Die dritte bis zehnte Spalte zeigt die Anzahl der koloniebildenden Einheiten (KBE) auf der LB-Agar-Platte für die einzelnen Verdünnungsstufen 10⁻¹ (1:10) bis 10⁻⁸ (1:10⁸). Die elfte Spalte zeigt die aus den Ergebnissen für die Verdünnungsstufen berechnete Bakterienkonzentration pro Milliliter Abwaschlösung im Zehnerlogarithmus. Der Wert von 7,4 für die erste Kontrollprobe entspricht demnach einer Bakterienkonzentration in der ursprünglichen Abwaschlösung dieser Probe von 10^{7,4}. Die zwölfte Spalte zeigt für die drei Kontrollproben den durch Mittelung berechneten Referenzwert 7,6 für die Log-Bakterienkonzentration. Für die übrigen Proben zeigt die zwölfte Spalte jeweils die Reduktion der jeweiligen Log-Bakterienkonzentration aus Spalte 11 gegenüber dem Referenzwert von 7,6 der Kontrollproben. Eine Log-Reduktion von 2,8 für die erste mit VE Wasser und Plasma behandelte Probe entspricht demnach einer Reduktion der Bakterienkonzentration in der zugehörigen Abwaschlösung um den Faktor 10^{2,8} (ca. 631).

Die Ergebnisse zeigen, dass die Beaufschlagung der Deckel mit einer Reinigungsflüssigkeit in Kombination mit der Plasmabeaufschlagung (bzw. der Arbeitsgasbeaufschlagung aus der Plasmaquelle) zu einer deutlich stärkeren Reduktion an koloniebildenden Einheiten führt als die Reinigungsflüssigkeit allein. Insbesondere wurden bereits bei geringen Wasserstoffperoxidkonzentrationen oder sogar mit reinem Wasser gute Desinfektionswirkungen erzielt.

Insbesondere zeigen die Ergebnisse die vorteilhafte Kombination der Beaufschlagung mit Wasserstoffperoxidlösung und Plasma (bzw. Arbeitsgas aus einer Plasmaquelle). So wurde bereits bei einer recht geringen Wasserstoffperoxidkonzentration von 3 Gew.-% durch die Kombination mit der Plasma- bzw. Arbeitsgasbeaufschlagung aus einer Plasmaquelle eine sehr gute Bakterienreduktion um mindestens 7,6-Logstufen erreicht, während die Beaufschlagung mit vollentsalztem (VE) Wasser und Plasma zum Teil nur zu geringeren Log-Reduktionen führte. Die bei der Kombination VE-Wasser und Plasma erreichte Log-Reduktion war jedoch immer noch deutlich besser als die praktisch nicht vorhandene Log-Reduktion bei Beaufschlagung mit z.B. 10%iger Wasserstoffperoxidlösung ohne anschließende Plasma- bzw. Arbeitsgasbehandlung.

**Tabelle 1**

| **Behandlung/ Treatment** | **Probe Nr./ sample nr.** | **KBE/ 100µl 10⁻¹** | **KBE/ 100µl 10⁻²** | **KBE/ 100µl 10⁻³** | **KBE/ 100µl 10⁻⁴** | **KBE/ 100µl 10⁻⁵** | **KBE/ 100µl 10⁻⁶** | **KBE/ 100µl 10⁻⁷** | **KBE/ 100µl 10⁻⁸** | **Log** | **Log Reduktion/ log reduction** |
|---|---|---|---|---|---|---|---|---|---|---|---|
| **Kontrolle** | 1 | >200 | >200 | 47 | 4 | 1 | - | - | - | 7,4 | 7,6 |
| | 2 | >200 | >200 | 50 | 6 | 2 | - | - | - | 7,6 | |
| | 3 | >200 | >200 | 60 | 16 | - | - | - | - | 7,7 | |
| **VEWasser mit Plasma** | 1 | 15 | - | - | - | - | - | - | - | 4,8 | 2,8 |
| | 2 | 6 | - | - | - | - | - | - | - | 4,4 | 3,2 |
| | 3 | - | - | - | - | - | - | - | - | 0 | 7,6 |
| **3% H₂O₂** | 1 | >200 | >200 | 103 | 26 | 3 | - | - | - | 8 | 0 |
| | 2 | >200 | >200 | 106 | 20 | 3 | - | - | - | 7,9 | 0 |
| **3% H2O2 mit Plasma** | 1 | - | - | - | - | - | - | - | - | 0 | 7,6 |
| | 2 | - | - | - | - | - | - | - | - | 0 | 7,6 |
| | 3 | - | - | - | - | - | - | - | - | 0 | 7,6 |
| **7% H2O2** | 1 | >200 | >200 | 102 | 20 | 2 | - | | - | 8,1 | 0 |
| | 2 | >200 | >200 | 133 | 35 | 3 | - | - | - | 8 | 0 |
| **7% H2O2 mit Plasma** | 1 | - | - | - | - | - | - | - | - | 0 | 7,6 |
| | 2 | - | - | - | - | - | - | - | - | 0 | 7,6 |
| | 3 | - | - | - | - | - | - | - | - | 0 | 7,6 |
| **10% H2O2** | 1 | >200 | >200 | 122 | 25 | 4 | - | - | - | 8 | 0 |
| | 2 | >200 | >200 | 99 | 26 | 4 | - | - | - | 8 | 0 |
| **10% H2O2 mit Plasma** | 1 | - | - | - | - | - | - | - | - | 0 | 7,6 |
| | 2 | - | - | - | - | - | - | - | - | 0 | 7,6 |
| | 3 | - | - | - | - | - | - | - | - | 0 | 7,6 |

Weiterhin wurden Versuche durchgeführt, die die Wirkung des Verfahrens aus Fig. 10 belegen.

Bei den Versuchen wurde ein kommerziell erhältliches Testsystem aus Bioindikator Sporenstreifen mit Geobacillus stearothermophilus 7953-Sporen und einer vorgegebenen Nährlösung verwendet. Konkret wurde das Produkt MesaStrip(R) Log6, erhältlich von Mesa Laboratories, Inc., Lakewood, CO, USA verwendet. Bei Geobacillus stearothermophilus handelt es sich um sehr robuste Sporen, die hohen Temperaturen und anderen widrigen Umgebungsbedingungen recht gut standhalten können.

Eine erste Gruppe von Sporenstreifen wurde nicht behandelt (Kontrollgruppe). Eine zweite Gruppe von Sporenstreifen wurde nur mit dem Arbeitsgas aus der in Fig. 8 dargestellten Plasmadüse 226 behandelt (Gruppe "Düse 1"). Eine dritte Gruppe von Sporenstreifen wurde nur mit dem Arbeitsgas aus der in Fig. 9 dargestellten Plasmaquelle 2' behandelt, wobei als Reinigungsflüssigkeit 272 Wasser verwendet wurde (Gruppe "Düse 2"). Eine vierte Gruppe von Sporenstreifen wurde schließlich entsprechend dem Verfahren aus Fig. 10 behandelt, d.h. mit beiden Plasmaquellen 226 und 2', wobei als Reinigungsflüssigkeit für die in Fig. 9 dargestellte Plasmaquelle 2' Wasser verwendet wurde (Gruppe "Düsen 1+2"). Anschließend wurden die Sporenstreifen entsprechend der Testvorschrift des Herstellers in die vorgegebene Nährlösung gegeben.

Das Testsystem ist herstellerseitig so vorbereitet, dass an einer Farbreaktion ablesbar ist, ob durch die vorige Behandlung der Sporenstreifen eine Reduktion der Keime um 6 Log-Stufen (d.h. um den Faktor 10⁶) erreicht wurde.

Die Ergebnisse der Versuche sind in der nachfolgenden Tabelle 2 zusammengefasst:

**Tabelle 2**

| | **Sporenstreifen-Gruppe** | **Reduktion um 6 Log-Stufen erreicht?** |
|---|---|---|
| **1** | Kontrollgruppe | Nein |
| **2** | Gruppe "Düse 1" | Nein |
| **3** | Gruppe "Düse 2" | Nein |
| **4** | Gruppe "Düsen 1+2" | Ja |

Wie aus Tabelle 2 ersichtlich wurde die Reduktion um 6 Log-Stufen nur bei den Proben erreicht, die mit dem in Fig. 10 beschriebenen Verfahren behandelt wurden. Eine Behandlung allein durch den Plasmastrahl aus der in Fig. 8 dargestellten Plasmadüse bzw. eine Behandlung allein durch das Arbeitsgas aus der in Fig. 9 dargestellten Plasmaquelle mit eingebrachtem Wasser als Reinigungsflüssigkeit, führte nicht zu einer Reduktion um 6 Log-Stufen.

Die Versuche zeigen, dass das in Fig. 10 beschriebene Verfahren besonders auch zur Desinfektion bei Vorliegen sehr robuster Keime geeignet ist.

## Patentansprüche

1. Verfahren zur Desinfektion von Komponenten einer Abfüllanlage (120, 180),
- bei dem die zu desinfizierenden Komponenten mit plasmaaktiviertem Wasser (70) beaufschlagt werden, wobei das plasmaaktivierte Wasser (70) zur Beaufschlagung auf die zu desinfizierenden Komponenten verdampft, versprüht und/oder zerstäubt wird.

2. Verfahren nach Anspruch 1,
**dadurch gekennzeichnet, dass** das plasmaaktivierte Wasser (70) mit einem Verfahren zur Herstellung von plasmaaktiviertem Wasser (70) hergestellt wird oder ist,
- bei dem in einem Entladungsraum (20) einer Plasmaquelle (2) ein atmosphärisches Plasma in einem Arbeitsgas erzeugt wird, vorzugsweise mittels dielektrisch behinderter Entladung, und
- bei dem das Arbeitsgas (22) aus dem Entladungsraum (20) über einen Tellerbelüfter (48) in ein Wasservolumen (46) eingeleitet wird.

3. Abfüllanlage, eingerichtet zum Abfüllen eines Produkts in Behälter,
- mit Beaufschlagungsmitteln (72, 150, 194) zum Beaufschlagen von Komponenten der Abfüllanlage (120, 180) mit einer Reinigungsflüssigkeit,
- wobei die Beaufschlagungsmittel dazu eingerichtet sind, die Komponenten mit plasmaaktiviertem Wasser (70) zu beaufschlagen, und
- wobei die Beaufschlagungsmittel (72, 150, 194)
- ein oder mehrere Düsen, die dazu eingerichtet sind, plasmaaktiviertes Wasser auf die zu desinfizierenden Komponenten zu sprühen, und/oder
- ein oder mehrere Verdampfer, die dazu eingerichtet sind, plasmaaktiviertes Wasser im Bereich der zu desinfizierenden Komponenten zu verdampfen, und/oder
- ein oder mehrere Zerstäuber, die dazu eingerichtet sind, plasmaaktiviertes Wasser im Bereich der zu desinfizierenden Komponenten zu zerstäuben,
umfassen,
**dadurch gekennzeichnet,**
- **dass** die Beaufschlagungsmittel (72, 150, 194) mit einem Reservoir verbunden sind, das plasmaaktiviertes Wasser (70) enthält, oder
- **dass** die Beaufschlagungsmittel (72, 150, 194) mit einer Vorrichtung (40) zur Herstellung von plasmaaktiviertem Wasser (70) verbunden sind.

4. Abfüllanlage nach Anspruch 3,
**dadurch gekennzeichnet, dass** eine Vorrichtung (40) zur Herstellung von plasmaaktiviertem Wasser (70) vorgesehen ist und dass die Beaufschlagungsmittel (72, 150, 194) derart an die Vorrichtung (40) zur Herstellung von plasmaaktiviertem Wasser (70) angeschlossen sind, dass von der Vorrichtung (40) zur Herstellung von plasmaaktiviertem Wasser (70) erzeugtes plasmaaktiviertes Wasser (70) zu den Beaufschlagungsmitteln (72, 150, 194) geleitet wird.

5. Abfüllanlage nach Anspruch 4,
**dadurch gekennzeichnet, dass** die Vorrichtung (40) einen Aktivierungsraum (44) zur Aufnahme eines Wasservolumens (46), einen Tellerbelüfter (48), der eine gasdurchlässige Membran (52) aufweist, die an den Aktivierungsraum (44) grenzt, und eine Plasmaquelle (2) zur Erzeugung eines atmosphärischen Plasmas in einem Arbeitsgas, aufweist, wobei die Plasmaquelle (2) eine Düsenöffnung (6) aufweist, aus der im Betrieb das Arbeitsgas (22) austritt und wobei die Plasmaquelle (2) derart an den Tellerbelüfter (48) angeschlossen ist, dass das im Betrieb aus der Düsenöffnung (6) austretende Arbeitsgas (22) durch die Membran (52) des Tellerbelüfters (48) in den Aktivierungsraum (44) gelangt.

6. Verfahren zur Desinfektion von Komponenten einer Abfüllanlage (120, 180) und/oder von beim Betrieb der Abfüllanlage (120, 180) verwendeten Materialien,
- bei dem die zu desinfizierenden Komponenten und/oder die zu desinfizierenden Materialien mit einer Reinigungsflüssigkeit (102) beaufschlagt werden,
- bei dem in einer Plasmaquelle (2), insbesondere mittels dielektrisch behinderter Entladung, ein atmosphärisches Plasma in einem Arbeitsgas erzeugt wird, das aus einer Düsenöffnung (6) der Plasmaquelle (2) austritt, und
- bei dem die mit der Reinigungsflüssigkeit (102) beaufschlagten Komponenten und/oder Materialien mit dem aus der Plasmaquelle (2) austretenden Arbeitsgas (22) beaufschlagt werden,
**dadurch gekennzeichnet,**
**dass** als Reinigungsflüssigkeit (102) eine wässrige Wasserstoffperoxid-Lösung mit einer Wasserstoffperoxid-Konzentration im Bereich von 1 bis 10 Gew.-% verwendet wird.

7. Verfahren nach Anspruch 6,
**dadurch gekennzeichnet, dass** das Arbeitsgas (22) aus der Düsenöffnung (6) in einen zumindest teilweise geschlossenen Raum (162, 206) eingeleitet wird, in dem sich die zu desinfizierenden Komponenten und/oder Materialien befinden.

## Claims

1. Method for the disinfection of components of a filling machine (120, 180),
- in which the components to be disinfected are exposed to plasma-activated water (70), the plasma-activated water (70) being vaporised, sprayed and/or atomised for exposure to the components to be disinfected.

2. Method according to claim 1,
**characterised in that** the plasma-activated water (70) is or has been is produced by a method for the production of plasma-activated water (70)
- in which, in a discharge chamber (20) of a plasma source (2), an atmospheric plasma is generated in a working gas, preferably by means of a dielectric barrier discharge, and
- in which the working gas (22) is introduced from the discharge chamber (20) via a disc diffuser (48) into a water volume (46).

3. Filling machine, configured for filling a product into containers,
- with exposure means (72, 150, 194) for exposing components of the filling machine (120, 180) to a cleaning liquid
- wherein the exposure means are configured to expose the components to plasma-activated water (70), and
- where the exposure means (72, 150, 194) comprise
- one or more nozzles configured to spray plasma-activated water onto the components to be disinfected, and/or
- one or more vaporisers configured to vaporise plasma-activated water in the area of the components to be disinfected, and/or
- one or more atomisers configured to atomise plasma-activated water in the area of the components to be disinfected,
**characterised**
- **in that** the exposure means (72, 150, 194) are connected to a reservoir containing plasma-activated water (70), or
- **in that** the exposure means (72, 150, 194) are connected to a device (40) for producing plasma-activated water (70).

4. Filling machine according to claim 3,
**characterised in that** a device (40) for producing plasma-activated water (70) is provided and **in that** the exposure means (72, 150, 194) are connected to the device (40) for producing plasma-activated water (70) in such a way that plasma-activated water (70) produced by the device (40) for producing plasma-activated water (70) is conducted to the exposure means (72, 150, 194).

5. Filling machine according to claim 4,
**characterised in that** the device (40) has an activation chamber (44) for receiving a volume of water (46), a disc diffusor (48) which has a gas-permeable membrane (52) adjoining the activation chamber (44), and a plasma source (2) for generating an atmospheric plasma in a working gas, wherein the plasma source (2) has a nozzle opening (6), from which the working gas (22) emerges during operation, and wherein the plasma source (2) is connected to the disc diffusor (48) in such a way that the working gas (22) emerging from the nozzle opening (6) during operation passes through the membrane (52) of the disc diffusor (48) into the activation chamber (44).

6. Method for disinfecting components of a filling machine (120, 180) and/or materials used in the operation of the filling machine (120, 180),
- in which the components to be disinfected and/or the materials to be disinfected are exposed to a cleaning liquid (102),
- in which, in a plasma source (2), an atmospheric plasma is generated in a working gas, in particular by means of dielectric barrier discharge, which working gas emerges from a nozzle opening (6) of the plasma source (2), and
- in which the components and/or materials exposed to the cleaning liquid (102) are exposed to the working gas (22) emerging from the plasma source (2),
**characterised**
**in that** an aqueous hydrogen peroxide solution with a hydrogen peroxide concentration in the range from 1 to 10 wt.% is used as the cleaning liquid (102).

7. Method according to claim 6,
**characterised in that** the working gas (22) is introduced from the nozzle opening (6) into an at least partially closed space (162, 206) in which the components and/or materials to be disinfected are located.

## Revendications

1. Procédé pour la désinfection de composants d'une installation de remplissage (120, 180),
- dans lequel les composants à désinfecter sont exposés à de l'eau activée par plasma (70), dans lequel l'eau activée par plasma (70) est vaporisée, pulvérisée et/ou nébulisée pour l'exposition des composants à désinfecter.

2. Procédé selon la revendication 1,
**caractérisé en ce que** l'eau activée par plasma (70) est/sera préparée avec un procédé pour la préparation d'eau activée par plasma (70),
- dans lequel, dans une chambre de décharge (20) d'une source de plasma (2), un plasma atmosphérique est produit dans un gaz de travail, de préférence au moyen d'une décharge entravée diélectriquement, et
- dans lequel le gaz de travail (22) issu de la chambre de décharge (20) est introduit dans un volume d'eau (46) par le biais d'un diffuseur (48).

3. Installation de remplissage, conçue pour le remplissage d'un produit dans des récipients,
- avec des moyens d'exposition (72, 150, 194) pour l'exposition de composants de l'installation de remplissage (120, 180) à un liquide de nettoyage,
- dans laquelle les moyens d'exposition sont conçus pour exposer les composants à de l'eau activée par plasma (70), et
- dans laquelle les moyens d'exposition (72, 150, 194) comportent
- une ou plusieurs buses, lesquelles sont conçues pour pulvériser de l'eau activée par plasma sur les composants à désinfecter, et/ou
- un ou plusieurs vaporisateurs, lesquels sont conçus pour vaporiser de l'eau activée par plasma dans la zone des composants à désinfecter, et/ou
- un ou plusieurs nébuliseurs, lesquels sont conçus pour nébuliser de l'eau activée par plasma dans la zone des composants à désinfecter,
**caractérisée en ce que**
- les moyens d'exposition (72, 150, 194) sont reliés à un réservoir qui contient l'eau activée par plasma (70), ou
- les moyens d'exposition (72, 150, 194) sont reliés à un dispositif (40) pour la préparation d'eau activée par plasma (70).

4. Installation de remplissage selon la revendication 3,
**caractérisée en ce qu'**il est prévu un dispositif (40) pour la préparation d'eau activée par plasma (70) et **en ce que** les moyens d'exposition (72, 150, 194) sont raccordés au dispositif (40) pour la préparation d'eau activée par plasma (70) de telle sorte que de l'eau activée par plasma (70) produite par le dispositif (40) pour la préparation d'eau activée par plasma (70) soit acheminée jusqu'aux moyens d'exposition (72, 150, 194).

5. Installation de remplissage selon la revendication 4,
**caractérisée en ce que** le dispositif (40) comprend une chambre d'activation (44) pour la réception d'un volume d'eau (46), un diffuseur (48), lequel comprend une membrane perméable aux gaz (52) qui est adjacente à la chambre d'activation (44), et une source de plasma (2) pour la production d'un plasma atmosphérique dans un gaz de travail, dans laquelle la source de plasma (2) comprend une ouverture de buse (6) de laquelle sort, en cours de fonctionnement, le gaz de travail (22), et dans laquelle la source de plasma (2) est raccordée au diffuseur (48) de telle sorte que le gaz de travail (22) sortant, en cours de fonctionnement, de l'ouverture de buse (6) passe à travers la membrane (52) du diffuseur (48) dans la chambre d'activation (44).

6. Procédé pour la désinfection de composants d'une installation de remplissage (120, 180) et/ou de matériel utilisé lors du fonctionnement de l'installation de remplissage (120, 180),
- dans lequel les composants à désinfecter et/ou le matériel à désinfecter sont exposés à un liquide de nettoyage (102),
- dans lequel, dans une source de plasma (2), en particulier au moyen d'une décharge entravée diélectriquement, un plasma atmosphérique est produit dans un gaz de travail qui sort d'une ouverture de buse (6) de la source de plasma (2), et
- dans lequel les composants et/ou le matériel exposés au liquide de nettoyage (102) sont exposés au gaz de travail (22) sortant de la source de plasma (2),
**caractérisé**
**en ce qu'**une solution aqueuse de peroxyde d'hydrogène avec une concentration en peroxyde d'hydrogène comprise dans la gamme allant de 1 à 10 % en poids est utilisée en tant que liquide de nettoyage (102).

7. Procédé selon la revendication 6,
**caractérisé en ce que** le gaz de travail (22) issu de l'ouverture de buse (6) est introduit dans une chambre au moins en partie fermée (162, 206) dans laquelle se trouvent les composants et/ou le matériel à désinfecter.
